# EUROPEAN PATENT APPLICATION

(11) **EP 3 450 547 A1**
(43) Date of publication of application: **06.03.2019**
(21) Application number: 17789652.9
(22) Date of filing: 27.04.2017
(51) Int. Cl.: C12N 5/0775

(54) **METHOD FOR PRODUCING ACTIVATED STEM CELLS**

(30) Priority: 27.04.2016 JP 2016088684; 26.04.2017 JP 2017087339
(71) Applicant: KintaroCellsPower Co., Ltd., Shinagawa-ku Tokyo 1410022 (JP)
(72) Inventor: GLADKOV, Alexei, Tokyo 108-0074 (JP)
(74) Representative: Ashton, Gareth Mark
(86) International application number: PCT/JP2017/016702
(87) International publication number: WO 2017/188370

(57) **Abstract**

[Problem] To provide a method for producing activated stem cells with which it is possible to efficiently grow a single type of dormant stem cells in a dormant state and to produce the necessary amount of a single type of activated stem cells having adequate activity.

[Solution] The method for producing activated stem cells has a dormant stem cell fixation step that injects a cultured product solution produced in the course of culturing a single type of dormant stem cells, a predetermined culture solution, and a single type of stem cells prior to storage of the dormant stem cells into a first culture vessel having a predetermined volume and a bottom surface of a predetermined area and fixes the dormant stem cells to the bottom surface of the first culture vessel, and a dormant stem cell culture step that cultures the dormant stem cells fixed to the bottom surface of the first culture vessel by the dormant stem cell fixation step, grows and activates the dormant stem cells until the total planar area of the dormant stem cells relative to the bottom surface area of the first culture vessel reaches a first target ratio, and changes the dormant stem cells into a single type of activated stem cells.

## Description

### TECHNICAL FIELD

The present invention relates to a method for producing activated stem cells that produces a single type of activated stem cell by activating a single type of dormant stem cell in a dormant state after storing for a predetermined period of time a single type of stem cell prepared by culturing a bone marrow liquid collected from a donor.

### BACKGROUND

Conventionally disclosed is a stem cell culture method including a culture medium for culturing a stem cell, and laser irradiation means for irradiating the entire culture medium with irradiation light of carbon dioxide gas laser having an irradiation energy of over 0 and 10 joules/cm² or less and a laser power density of 0.1W/cm² or less to activate stem cells in the medium, by defocus of the irradiation energy to a low power of 0.1 or more and 2.5 joules/cm² or less, and the method activates such stem cells by low-power laser irradiation and subsequently proliferates the stem cells to a target number for a predetermined rest period. The stem cell culture method can activate and significantly proliferate stem cells present in a tissue sampled from a human or nonhuman (animal), or a cell thereof.

### PRIOR ART DOCUMENTS

### PATENT DOCUMENTS

Patent Document 1: JP-A-2015-186465

### SUMMARY OF THE INVENTION

### PROBLEM TO BE SOLVED BY THE INVENTION

Stem cells, once proliferated by the stem cell culture method and other stem cell culture methods, are stored in a refrigerator or a freezer, placed at a predetermined temperature for a predetermined period of time, and taken out of the refrigerator or the freezer for use. This low-temperature preservation provides dormant stem cells in a dormant state with low activity, thereby inevitably subjecting such dormant stem cells to an activation process, which leads only some dormant stem cells to survive and the other residual stem cells to die. The resulting dead dormant stem cells may prevent proliferation of surviving dormant stem cells, thereby failing to efficiently proliferate dormant stem cells and produce adequately activated stem cells in required volume from thawed dormant stem cells.

Since various types of stem cells are present in a tissue or its cells collected from a donor, culturing such stem cells leads to their own proliferation, and the culture process fails to culture only a specific single type of stem cell, thereby unsuccessfully producing a single type of activated stem cell by proliferating and activating a single type of dormant stem cell. In fact, activated stem cells are generally used in each type of disease treatment (e.g. for cardiovascular diseases and central nervous system diseases), regenerative medicine and nontherapeutic applications, however, when cultured as those containing various types, the resulting activated stem cells provide smaller effects in each type of disease treatment and regenerative medicine than only a single and specific type of activated stem cell.

The present invention has an object to provide a method for producing activated stem cells capable of efficiently proliferating a single type of dormant stem cell in a dormant state and producing a single type of activated stem cell having adequate activity in required volume. The present invention has another object to provide a method for producing activated stem cells capable of producing a single type of activated stem cell containing no various types of activated stem cells and having more significant effects in each type of disease treatment and regenerative medicine.

### MEANS FOR SOLVING THE PROBLEM

To solve these problems, the present invention provides a method for producing activated stem cells that produces a single type of activated stem cell by activating a single type of dormant stem cell in a dormant state after storing for a predetermined period of time a single type of stem cell prepared by culturing a bone marrow liquid collected from a donor.

The method for producing activated stem cells according to the present invention includes: fixing a dormant stem cell for feeding the single type of dormant stem cell, a predetermined culture liquid, and a culture product liquid prepared in the process of culturing the single type of stem cell before storing the dormant stem cell into a first culture container having a predetermined capacity and a bottom surface of a predetermined area, and fixing the dormant stem cell to the bottom of the first culture container; and culturing a dormant stem cell for culturing the dormant stem cell fixed to the bottom of the first culture container by the step of fixing a dormant stem cell, proliferating and activating the dormant stem cell until the total area of the dormant stem cell reaches a first target ratio of the bottom-surface area of the first culture container, and transforming the dormant stem cell into the single type of activated stem cell.

As one example of the present invention, in the step of culturing a dormant stem cell, a mixed culture liquid of the culture liquid and the culture product liquid is discharged from the first culture container after fixing the dormant stem cell to the bottom of the first culture container by the step of fixing a dormant stem cell, a new culture liquid and a new culture product liquid are fed into the first culture container, and the dormant stem cell fixed to the bottom of the first culture container is cultured, using a new mixed culture liquid of a new culture liquid and a new culture product liquid.

As another example of the present invention, in the step of fixing a dormant stem cell, the first culture container is allowed to statically stand at approximately the same temperature as the body temperature at a predetermined tilted angle for 12 to 24 hours, the deformation of a dormant stem cell from the initial plane shape in the first culture container is observed at an interval of approx. 1 to 2 hours for the period of 12 to 24 hours, and when the dormant stem cell is deformed from the initial plane shape to a predetermined plane shape, it is determined that the dormant stem cell is fixed to the bottom surface of the first culture container.

As another example of the present invention, the initial plane shape of the dormant stem cell is an approximately circular shape, and the plane shape of the dormant stem cell deformed is primarily the approximately circular shape, and a flat shape when the dormant stem cell elongates in amorphous form in one direction, and in the step of fixing a dormant stem cell, when the dormant stem cell is deformed to the amorphous flat shape, it is determined that the dormant stem cell is fixed to the bottom surface of the first culture container.

As another example of the present invention, the first target ratio of the total area of the dormant stem cell is 70 to 80% of the bottom-surface area of the first culture container, and in the step of culturing a dormant stem cell, the first culture container is allowed to statically stand at approximately the same temperature as the body temperature at a predetermined tilted angle for 36 to 48 hours, and the total area of the dormant stem cell fixed to the bottom surface of the first culture container in relation to the bottom-surface area of the first culture container is observed at an interval of approx. 1 to 2 hours for the period of 36 to 48 hours.

As another example of the present invention, the method for producing activated stem cells includes: fixing an activated stem cell for extracting from the first culture container the activated stem cell when the total area of the dormant stem cell reaches the first target ratio of the bottom-surface area of the first culture container, feeding the activated stem cell extracted, a new culture liquid, and a new culture product liquid into a second culture container having a predetermined capacity and a bottom surface of a predetermined area, the capacity being larger than the first culture container, and fixing the activated stem cell to the bottom of the second culture container; and culturing an activated stem cell for culturing the activated stem cell fixed to the bottom of the second culture container by the step of fixing an activated stem cell, and proliferating the activated stem cell until the total area of the activated stem cell reaches a second target ratio of the bottom-surface area of the second culture container.

As another example of the present invention, in the step of culturing an activated stem cell, a mixed culture liquid of the culture liquid and the culture product liquid is discharged from the second culture container after fixing the activated stem cell to the bottom of the second culture container by the step of fixing an activated stem cell, a new culture liquid and a new culture product liquid are fed into the second culture container, and the activated stem cell fixed to the bottom of the second culture container is cultured, using a new mixed culture liquid of a new culture liquid and a new culture product liquid.

As another example of the present invention, in the step of fixing an activated stem cell, the second culture container is allowed to statically stand at approximately the same temperature as the body temperature at a predetermined tilted angle for 36 to 48 hours, the deformation of the activated stem cell from the initial plane shape in the second culture container is observed at an interval of approx. 1 to 2 hours for the period of 36 to 48 hours, and when the activated stem cell is deformed from the initial plane shape to a predetermined plane shape, it is determined that the activated stem cell is fixed to the bottom surface of the second culture container.

As another example of the present invention, the initial plane shape of the activated stem cell is an approximately circular shape, and the plane shape of the activated stem cell deformed is primarily the approximately circular shape, and a flat shape when the activated stem cell elongates in amorphous form in one direction, and in the step of fixing an activated stem cell, when the activated stem cell is deformed to the amorphous flat shape, it is determined that the activated stem cell is fixed to the bottom surface of the second culture container.

As another example of the present invention, the second target ratio of the total area of the activated stem cell is 88 to 92% of the bottom-surface area of the second culture container, and in the step of culturing an activated stem cell, the second culture container is allowed to statically stand at approximately the same temperature as the body temperature at a predetermined tilted angle for 36 to 48 hours, and the total area of the activated stem cell fixed to the bottom surface of the second culture container in relation to the bottom-surface area of the second culture container is observed at an interval of approx. 1 to 2 hours for the period of 36 to 48 hours.

As another example of the present invention, the culture product liquid, in the process of culturing the single type of stem cell, contains a predetermined metabolite secreted from the single type of stem cell.

As another example of the present invention, the single type of stem cell is prepared by the steps of: first fixing a stem cell for separating in layers a bone marrow liquid collected from the donor, extracting an intermediate-layer bone marrow liquid positioned in an intermediate layer of the bone marrow liquid separated in layers, feeding the intermediate-layer bone marrow liquid and a predetermined culture liquid into a third culture container having a predetermined capacity and a bottom surface of a predetermined area, and fixing a first stem cell contained in the intermediate-layer bone marrow liquid to the bottom surface of the third culture container; first culturing a stem cell for discharging the culture liquid in the third culture container after fixing the first stem cell to the bottom surface of the third culture container by the step of first fixing a stem cell, feeding a new culture liquid into the third culture container, culturing the first stem cell, and proliferating the first stem cell until the total area of the first stem cell reaches a third target ratio of the bottom-surface area of the third culture container; second fixing a stem cell for centrifugally separating in layers the first stem cell cultured by the step of first culturing a stem cell, extracting a second stem cell positioned in the lowermost layer of the first stem cell separated in layers, feeding the second stem cell and a new culture liquid into a fourth culture container having a predetermined capacity and a bottom surface of a predetermined area, the capacity being larger than the third culture container, and fixing the second stem cell to the bottom of the fourth culture container; and second culturing a stem cell for discharging a culture liquid in the fourth culture container after fixing the second stem cell to the bottom of the fourth culture container by the step of second fixing a stem cell, feeding a new culture liquid into the fourth culture container, culturing the second stem cell, and proliferating the second stem cell until the total area of the second stem cell reaches a fourth target ratio of the bottom-surface area of the fourth culture container.

As another example of the present invention, the culture product liquid is a residual culture liquid obtained by extracting the single second stem cell from the fourth culture container.

As another example of the present invention, these stem cells are mesenchymal stem cells.

### EFFECT OF THE INVENTION

The method for producing activated stem cells according to the present invention feeds a culture product liquid prepared in the process of culturing a single type of stem cell before storing a dormant stem cell to encourage the fixation and proliferation of a single type of dormant stem cell to the bottom of the first culture container, thereby immediately fixing the dormant stem cell to the bottom of the first culture container, using its culture product liquid, immediately proliferating the dormant stem cell until the total area reaches a first target ratio, using its culture product liquid, efficiently and assuredly proliferating a single type of dormant stem cell in a dormant state, and efficiently producing a single type of activated stem cell having adequate activity in required volume from the dormant stem cell. The method for producing activated stem cells can produce a single type of pure (clear) activated stem cell containing no undesired stem cell and various types of stem cells are not contained, thereby allowing production of an activated stem cell having significant effects in each type of disease treatment and regenerative medicine, capable of effective use in treating each type of disease in an appropriate and timely manner, and capable of effective use in regenerating each type of tissue or organ in an appropriate and timely manner.

The method for producing activated stem cells, which discharges a mixed culture liquid of a culture liquid and a culture product liquid from a first culture container after fixing a dormant stem cell to the bottom of the first culture container, feeds a new culture liquid and a new culture product liquid into the first culture container, and cultures the dormant stem cell fixed to the bottom of the first culture container, using a new mixed culture liquid of a new culture liquid and a new culture product liquid, assuredly encourages the proliferation of the dormant stem cell by a new culture product liquid prepared in the process of culturing a single type of stem cell before storing a dormant stem cell, thereby allowing immediate proliferation of a dormant stem cell until the total area reaches a first target ratio, using a new culture product liquid, efficient and assured proliferation of a single type of dormant stem cell in a dormant state, and efficient production of a single type of activated stem cell having adequate activity in required volume. The method for producing activated stem cells can produce a single type of pure (clear) activated stem cell containing no undesired stem cell, and various types of stem cells are not contained, thereby allowing production of an activated stem cell having significant effects in each type of disease treatment and regenerative medicine, capable of effective use in treating each type of disease in an appropriate and timely manner, and capable of effective use in regenerating each type of tissue or organ in an appropriate and timely manner.

The method for producing activated stem cells, in which a first culture container is allowed to statically stand at approximately the same temperature as the body temperature at a predetermined tilted angle for 12 to 24 hours, the deformation of a dormant stem cell from the initial plane shape in the first culture container is observed at an interval of approx. 1 to 2 hours for the period of 12 to 24 hours, and when the dormant stem cell is deformed from the initial plane shape to a predetermined plane shape, it is determined that the dormant stem cell is fixed to the bottom surface of the first culture container, can assuredly fix the dormant stem cell to the bottom of the first culture container by allowing the first culture container to statistically stand at a predetermined tilted angle for 12 to 24 hours and assuredly encourage the proliferation of the dormant stem cell. The method for producing activated stem cells, in which the deformation of the dormant stem cell from the initial plane shape in the first culture container is observed at an interval of approx. 1 to 2 hours for the period of 12 to 24 hours to properly confirm the fixation of the dormant stem cell to the bottom of the first culture container, can assuredly confirm the fixation of the dormant stem cell and assuredly proliferate the dormant stem cell to produce a single type of activated stem cell having adequate activity.

The method for producing activated stem cells, in which the initial plane shape of a dormant stem cell is an approximately circular shape, and the plane shape of the dormant stem cell deformed is primarily the approximately circular shape, and a flat shape when the dormant stem cell elongates in amorphous form in one direction, and in the step of fixing a dormant stem cell, when the dormant stem cell is deformed to the amorphous flat shape, it is determined that the dormant stem cell is fixed to the bottom surface of a first culture container, observes the deformation of the dormant stem cell from the approximately circular shape to the plane shape on the bottom of the first culture container to properly determine the fixation of the dormant stem cell to the bottom of the first culture container, thereby allowing assured confirmation of the fixation of the dormant stem cell in the first culture container and assured proliferation of the dormant stem cell to produce a single type of activated stem cell having adequate activity.

The method for producing activated stem cells, in which the first target ratio of the total area of a dormant stem cell is 70 to 80% of the bottom-surface area of a first culture container, and in the step of culturing a dormant stem cell, the first culture container is allowed to statically stand at approximately the same temperature as the body temperature at a predetermined tilted angle for 36 to 48 hours, and the total area of the dormant stem cell fixed to the bottom surface of the first culture container in relation to the bottom-surface area of the first culture container is observed at an interval of approx. 1 to 2 hours for the period of 36 to 48 hours, allows the first culture container to statistically stand at a predetermined tilted angle for 36 to 48 hours to assuredly encourage the proliferation of the dormant stem cell fixed to the bottom of the first culture container. The method for producing activated stem cells observes the total area at an interval of approx. 1 to 2 hours to properly confirm the total area of the dormant stem cell in relation to the bottom-surface area of the first culture container, thereby allowing assured confirmation of the proliferation of the dormant stem cell in the first culture container and assured proliferation of the dormant stem cell to produce a single type of activated stem cell having adequate activity. The method for producing activated stem cells, when the total area of the dormant stem cell in relation to the bottom-surface area of the first culture container exceeds 80% to proliferate a dormant stem cell, gradually reduces the activity of the dormant stem cell, and when the total area of the first stem cell in relation to the bottom-surface area of the first culture container increases to 70 to 80% for proliferation, extracts the dormant stem cell activated from the first culture container, thereby maintaining the activity of the dormant stem cell activated to produce a single type of activated stem cell having adequate activity from a dormant stem cell.

The method for producing activated stem cells, which extracts an activated stem cell from a first culture container when the total area of a dormant stem cell reaches a first target ratio of the bottom-surface area of the first culture container, feeds an activated stem cell extracted, a new culture liquid, and a new culture product liquid into a second culture container having a predetermined capacity and a bottom surface of a predetermined area, the capacity being larger than the first culture container, fixes the activated stem cell to the bottom of the second culture container, cultures the activated stem cell fixed to the bottom of the second culture container, and proliferates the activated stem cell until the total area of the activated stem cell reaches a second target ratio of the bottom-surface area of the second culture container, encourages the fixation of an activated stem cell to the bottom of the second culture container and proliferation of the activated stem cell by a culture product liquid prepared in the process of culturing a single type of stem cell before storing an activated stem cell (dormant stem cell), thereby allowing immediate fixation of the activated stem cell to the bottom of the second culture container, using its culture product liquid, immediate proliferation of the activated stem cell until the total area reaches a second target ratio, using its culture product liquid, efficient and assured proliferation of a single type of activated stem cell, and efficient production of a single type of activated stem cell having adequate activity in required volume. The method for producing activated stem cells can produce a single type of pure (clear) activated stem cell containing no undesired stem cell, and various types of stem cells are not contained, thereby allowing production of an activated stem cell having significant effects in each type of disease treatment and regenerative medicine, capable of effective use in treating each type of disease in an appropriate and timely manner, and capable of effective use in regenerating each type of tissue or organ in an appropriate and timely manner.

The method for producing activated stem cells, which discharges a mixed culture liquid of a culture liquid and a culture product liquid from a second culture container after fixing an activated stem cell to the bottom of the second culture container, feeds a new culture liquid and a new culture product liquid into the second culture container, and cultures the activated stem cell fixed to the bottom of the second culture container, using a new mixed culture liquid of a new culture liquid and a new culture product liquid, assuredly encourages the proliferation of an activated stem cell by a new culture product liquid prepared in the process of culturing a single type of stem cell before storing an activated stem cell (dormant stem cell), allowing immediate proliferation of the activated stem cell until the total area reaches a second target ratio, using a new culture product liquid, efficient and assured proliferation of a single type of activated stem cell, and efficient production of a single type of activated stem cell having adequate activity in required volume. The method for producing activated stem cells can produce a single type of pure (clear) activated stem cell containing no undesired stem cell, and various types of stem cells are not contained, thereby allowing production of an activated stem cell having significant effects in each type of disease treatment and regenerative medicine, capable of effective use in treating each type of disease in an appropriate and timely manner, and capable of effective use in regenerating each type of tissue or organ in an appropriate and timely manner.

The method for producing activated stem cells, in which a second culture container is allowed to statically stand at approximately the same temperature as the body temperature at a predetermined tilted angle for 36 to 48 hours, the deformation of an activated stem cell from the initial plane shape in the second culture container is observed at an interval of approx. 1 to 2 hours for the period of 36 to 48 hours, and when the activated stem cell is deformed from the initial plane shape to a predetermined plane shape, it is determined that the activated stem cell is fixed to the bottom surface of the second culture container, can assuredly fix the activated stem cell to the bottom of the second culture container by allowing the second culture container to statistically stand at a predetermined tilted angle for 36 to 48 hours and assuredly encourage the proliferation of the activated stem cell. The method for producing activated stem cells, in which the deformation of the activated stem cell from the initial plane shape in the second culture container is observed at an interval of approx. 1 to 2 hours for the period of 36 to 48 hours to properly confirm the fixation of the activated stem cell to the bottom of the second culture container, can assuredly confirm the fixation of the activated stem cell and assuredly proliferate the activated stem cell to produce a single type of activated stem cell having adequate activity.

The method for producing activated stem cells, in which the initial plane shape of an activated stem cell is an approximately circular shape, and the plane shape of the activated stem cell deformed is primarily the approximately circular shape, and a flat shape when the activated stem cell elongates in amorphous form in one direction, and when the activated stem cell is deformed to the amorphous flat shape, it is determined that the activated stem cell is fixed to the bottom surface of the second culture container, observes the deformation of the activated stem cell from the approximately circular shape to the plane shape on the bottom of the second culture container to properly determine the fixation of the activated stem cell to the bottom of the second culture container, can assuredly confirm the fixation of the activated stem cell in the second culture container and assuredly proliferate the activated stem cell to produce a single type of activated stem cell having adequate activity.

The method for producing activated stem cells, in which the second target ratio of the total area of the activated stem cell is 88 to 92% of the bottom-surface area of the second culture container, and the second culture container is allowed to statically stand at approximately the same temperature as the body temperature at a predetermined tilted angle for 36 to 48 hours, and the total area of the activated stem cell fixed to the bottom surface of the second culture container in relation to the bottom-surface area of the second culture container is observed at an interval of approx. 1 to 2 hours for the period of 36 to 48 hours, allows the second culture container to statistically stand at a predetermined tilted angle for 36 to 48 hours to assuredly encourage the proliferation of the activated stem cell fixed to the bottom of the second culture container. The method for producing activated stem cells observes the total area at an interval of approx. 1 to 2 hours to properly confirm the total area of the activated stem cell in relation to the bottom-surface area of the second culture container, thereby allowing assured confirmation of the proliferation of the activated stem cell in the second culture container and assured proliferation of the activated stem cell to produce a single type of activated stem cell having adequate activity. The method for producing activated stem cells, when the total area of the activated stem cell in relation to the bottom-surface area of the second culture container exceeds 92% to proliferate the activated stem cell, gradually reduces the activity of the activated stem cell, and when the total area of the second stem cell in relation to the bottom-surface area of the second culture container increases to 88 to 92% for proliferation, extracts the activated stem cell from the second culture container, thereby maintaining the activity of the activated stem cell to produce a single type of activated stem cell having adequate activity.

The method for producing activated stem cells, in which the culture product liquid contains a predetermined metabolite secreted from a single type of stem cell in the process of culturing the single type of stem cell, using a culture product liquid containing a predetermined metabolite secreted from the single type of stem cell, a metabolite of its stem cell itself triggers immediate activation of a dormant stem cell or an activated stem cell. Accordingly, the method for producing activated stem cells encourages the fixation of a dormant stem cell to the bottom of the first culture container and the fixation of an activated stem cell to the bottom of the second culture container and proliferation of a dormant stem cell in the first culture container and the proliferation of an activated stem cell in the second culture container, thereby allowing immediate fixation of the dormant stem cell or the activated stem cell to the bottom of the culture container, using its culture product liquid, immediate proliferation of the dormant stem cell or the activated stem cell until the total area reaches a target ratio, using its culture product liquid, efficient and assured proliferation of a single type of dormant stem cell in a dormant state or activated stem cell, and efficient production of a single type of activated stem cell having adequate activity in required volume.

The method for producing activated stem cells, which prepares a single type of stem cell in the steps of: extracting an intermediate-layer bone marrow liquid positioned in an intermediate layer of the bone marrow liquid separated in layers, and fixing a first stem cell contained in the intermediate-layer bone marrow liquid to the bottom surface of the third culture container; culturing the first stem cell, and proliferating the first stem cell until the total area of the first stem cell reaches a third target ratio of the bottom-surface area of the third culture container; extracting a second stem cell positioned in the lowermost layer of the first stem cell separated in layers, and fixing the second stem cell to the bottom of a larger fourth culture container; culturing the second stem cell, and proliferating the second stem cell until the total area of the second stem cell reaches a fourth target ratio of the bottom-surface area of the fourth culture container, contains no various types of stem cells, thereby allowing production of a single type of a pure (clear) activated stem cell containing no undesired stem cell having significant effects in each type of disease treatment and regenerative medicine, capable of effective use in treating each type of disease in an appropriate and timely manner, and capable of effective use in regenerating each type of tissue or organ in an appropriate and timely manner.

The method for producing activated stem cells, in which the culture product liquid is a residual culture liquid obtained by extracting a single second stem cell from a fourth culture container, allows a residual culture liquid obtained by extracting the second stem cell to contain a predetermined metabolite secreted from the single type of stem cell, and a metabolite of its stem cell triggers immediate activation of a dormant stem cell or an activated stem cell. Accordingly, the method for producing activated stem cells encourages the fixation of the dormant stem cell to the bottom of the first culture container and the fixation of the activated stem cell to the bottom of the second culture container and proliferation of the dormant stem cell in the first culture container and the proliferation of the activated stem cell in the second culture container, thereby allowing immediate fixation of the dormant stem cell or the activated stem cell to the bottom of the culture container, using its culture product liquid, immediate proliferation of the dormant stem cell or the activated stem cell until the total area reaches a target ratio, using its culture product liquid, efficient and assured proliferation of a single type of dormant stem cell in a dormant state or an activated stem cell, and efficient production of a single type of activated stem cell having adequate activity in required volume.

The method for producing activated stem cells, in which such stem cells are mesenchymal stem cells, can efficiently and assuredly proliferate a single type of mesenchymal dormant stem cell in a dormant state or a mesenchymal activated stem cell to efficiently produce a single type of mesenchymal activated stem cell having adequate activity in required volume. The method for producing activated stem cells can produce a single type of pure (clear) activated stem cell containing no undesired stem cell, and various types of stem cells are not contained, thereby allowing production of an activated stem cell having significant effects in each type of disease treatment and regenerative medicine, capable of effective use in treating each type of disease in an appropriate and timely manner, and capable of effective use in regenerating each type of tissue or organ in an appropriate and timely manner.

### BRIEF DESCRIPTION OF THE DRAWINGS

FIG. 1 is a schematic block diagram illustrating one example of an activated stem cell culturing system;
FIG. 2 is an explanatory drawing illustrating one example of a step of fixing a dormant stem cell;
FIG. 3 is a side view illustrating a first flat culture container;
FIG. 4 is a partially enlarged view illustrating one example of a plane shape of a dormant stem cell;
FIG. 5 is a partially enlarged view illustrating another example of a plane shape of a dormant stem cell;
FIG. 6 is a partially enlarged view illustrating another example of a plane shape of a dormant stem cell;
FIG. 7 is an explanatory drawing illustrating one example of a step of fixing an activated stem cell;
FIG. 8 is a side view illustrating a second flat culture container;
FIG. 9 is a partially enlarged view illustrating another example of a plane shape of an activated stem cell;
FIG. 10 is a partially enlarged view illustrating another example of a plane shape of an activated stem cell;
FIG. 11 is a partially enlarged view illustrating another example of a plane shape of an activated stem cell;
FIG. 12 is a perspective view illustrating a glass test tube used in a step of first fixing a stem cell;
FIG. 13 is an explanatory drawing illustrating a step of first fixing a stem cell following the step in FIG. 10;
FIG. 14 is an explanatory drawing illustrating a step of first fixing a stem cell following the step in FIG. 11;
FIG. 15 is a partially enlarged view illustrating one example of a plane shape of a stem cell;
FIG. 16 is a partially enlarged view illustrating another example of a plane shape of a stem cell;
FIG. 17 is a partially enlarged view illustrating another example of a plane shape of a stem cell;
FIG. 18 is a perspective view illustrating a glass test tube and a centrifugal separator used in a step of second fixing a stem cell;
FIG. 19 is a perspective view illustrating a glass test tube after centrifugal separation;
FIG. 20 is a partially enlarged view illustrating one example of a plane shape of a stem cell (second stem cell);
FIG. 21 is a partially enlarged view illustrating another example of a plane shape of a stem cell (second stem cell);
FIG. 22 is a partially enlarged view illustrating another example of a plane shape of a stem cell (second stem cell); and
FIG. 23 is a diagram illustrating one example of a stem cell (second stem cell) and a culture product liquid stored.

### DESCRIPTION OF THE PREFERRED EMBODIMENTS

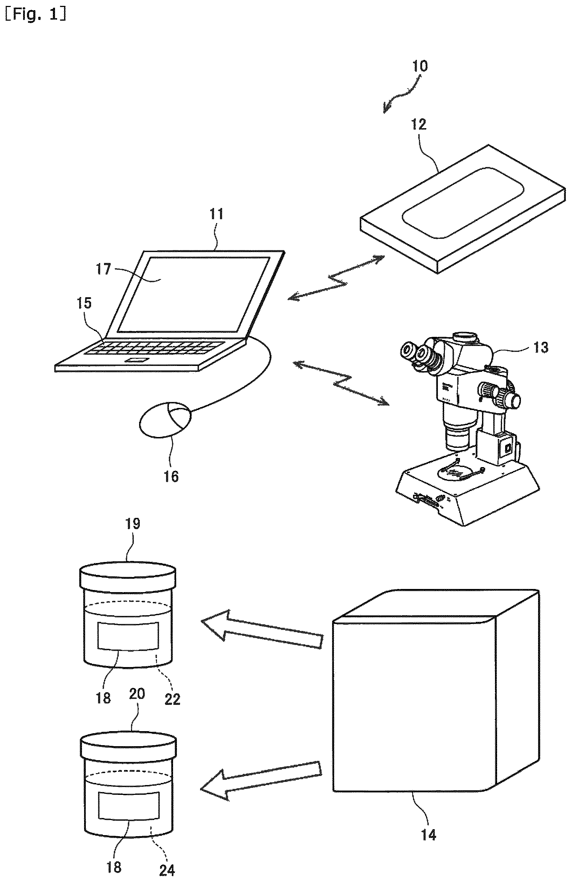

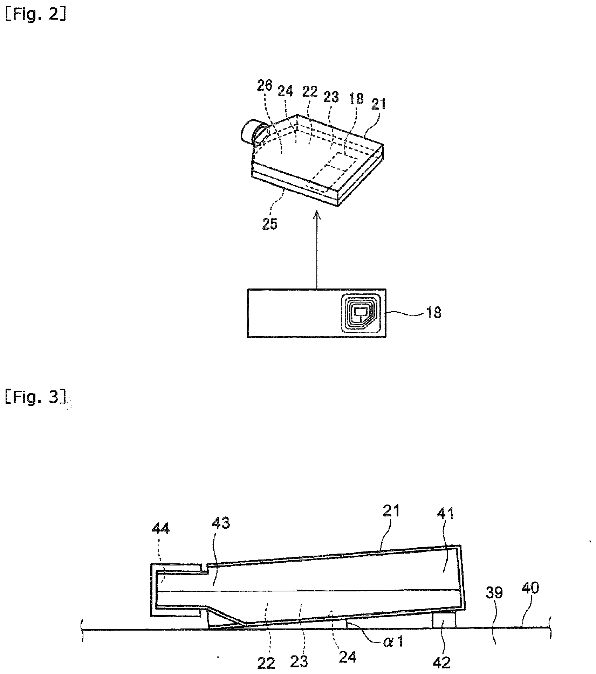

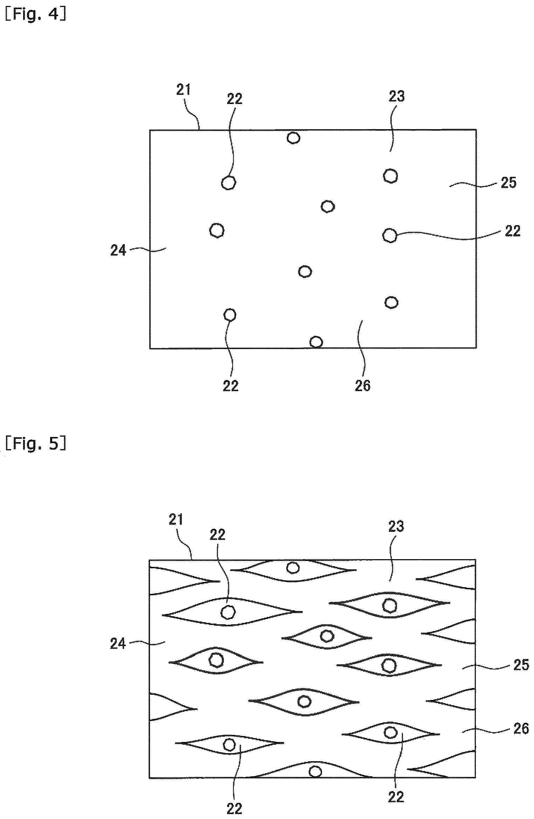

With reference to the accompanying drawings such as FIG. 1 illustrating a schematic block diagram of one example of an activated stem cell culturing system 10, the method for producing a single and specific type of mesenchymal activated stem cell according to the present invention will be described as follows. FIG. 2 is an explanatory drawing illustrating one example of a step of fixing a dormant stem cell, and FIG. 3 is a side view illustrating a first flat culture container 21. FIG. 4 is a partially enlarged view illustrating one example of a plane shape of a dormant stem cell 22, and FIG. 5 is a partially enlarged view illustrating another example of a plane shape of a dormant stem cell 22. FIGS. 4 and 5 illustrate magnified views of a plane shape of a dormant stem cell 22 imaged with an electron microscope 13.

A single type of mesenchymal activated stem cell 27 is obtained by activating a mesenchymal dormant stem cell 22 in a dormant state after storing for a predetermined period of time a single and specific type of mesenchymal stem cell 30 prepared by culturing a bone marrow liquid 29 collected from a donor. The activated stem cell 27 is produced by subjecting the dormant stem cell 22 in a dormant state to a step of fixing a dormant stem cell and a step of culturing a dormant stem cell, using a culture product liquid 24 prepared in the process of culturing the stem cell 30 as an ingredient of the dormant stem cell 22 before storing the dormant stem cell 22.

Also, the activated stem cell is prepared by subjecting the dormant stem cell 22 in a dormant state to a step of fixing a dormant stem cell and a step of culturing a dormant stem cell, as well as a step of fixing an activated stem cell and a step of culturing an activated stem cell, using a culture product liquid 24 prepared in the process of culturing the stem cell 30 as an ingredient of the dormant stem cell 22 before storing the dormant stem cell 22. The stem cell 30 before being stored and the dormant stem cell 22 after being stored are the same stem cells.

The culture product liquid 24 contains a predetermined metabolite secreted from a single type of stem cell 30 in the process of culturing the single type of stem cell 30. Using the culture product liquid 24 containing a predetermined metabolite secreted from the single type of stem cell 30, a metabolite of its stem cell 30 itself triggers immediate activation of a dormant stem cell 22 and an activated stem cell 27. Accordingly, the culture product liquid 24 encourages the fixation of the dormant stem cell 22 and the activated stem cell 27 and proliferation of the dormant stem cell 22 and the activated stem cell 27, thereby allowing immediate fixation of the dormant stem cell 22 or the activated stem cell 27, and using its culture product liquid 24, immediate proliferation of the dormant stem cell 22 or the activated stem cell 27, and using its culture product liquid 24, efficient and assured proliferation of the dormant stem cell 22 or the activated stem cell 27.

The activated stem cell culturing system 10 is provided with a computer 11, an IC tag reader/writer 12, an electron microscope 13, and a refrigerator 14 or a freezer 14. The computer 11, including a central processing unit (CPU or virtual CPU), a storage unit (memory or virtual memory), and a mass storage area (including hard disk or virtual hard disk), is operated by a physical OS (operating system) and a virtual OS (virtual operating system).

To the computer 11 are connected input devices such as a keyboard 15 and a mouse 16 and output devices such as a display 17 and a printer (not shown) via an interface (wireless or wired). The IC tag reader/writer 12 and the electron microscope 13 are connected to the computer 11 via an interface (wireless or wired). The electron microscope 13 includes an imaging function for allowing an image sensor to image a magnified view of a subject and an image transmitting function for transmitting the magnified view to the computer 11.

The activated stem cell culturing system 10 employs an IC tag 18 to manage donor data (donor specific information), as well as stem cell data regarding a stem cell, a dormant stem cell 22, and an activated stem cell. The donor data includes donor's name, permanent address, telephone number, date of birth, gender, blood type, height, weight, and e-mail address, and the donor data is stored in an IC tag so as to be associated with a donor identifier. The stem cell data includes stem cell specific information, stem cell production date, culture product liquid production date, and culture product liquid specific information, and the stem cell data is stored in an IC tag 18 so as to be associated with a donor identifier and a stem cell identifier.

A single and specific type of stem cell 30 prepared by culturing a bone marrow liquid 29 is placed in a stem cell storing container 19, and stored in a refrigerator 14 or freezer 14 at a predetermined temperature (3 to 5°C or refrigerated) for a predetermined period of time. On the outer peripheral surface of the stem cell storing container 19 that stores the stem cell 30 is affixed an IC tag 18 that stores donor data and stem cell data.

The culture product liquid 24 prepared in the process of culturing the stem cell 30 as an ingredient of an activated stem cell 27 (dormant stem cell 22) is placed in a product liquid storing container 20, and stored in a refrigerator 14 or freezer 14 at a predetermined temperature for a predetermined period of time. On the outer peripheral surface of the product liquid storing container 20 that stores a culture product liquid 24 is affixed an IC tag 18 that stores donor data and stem cell data.

When the system 10 is activated on the computer 11, an initial screen (not shown) is displayed on a display 17. The initial screen displays a stem cell culture button, a dormant stem cell culture button, an activated stem cell culture button, and a log-out button. Those in charge such as doctors, nurses, and researchers click the dormant stem cell culture button displayed on the display 17. When the dormant stem cell culture button is clicked, the computer 11 displays a data comparison button on the display 17.

In a step of fixing a dormant stem cell, those in charge click the data comparison button, take the stem cell storing container 19 or the product liquid storing container 20 out of the refrigerator 14 or the freezer 14, and allow the IC tag reader/writer 12 to read the data of the IC tag 18 affixed to the stem cell storing container 19 or the product liquid storing container 20. The computer 11 compares a donor identifier or a stem cell identifier of the IC tag 18 of the stem cell storing container 19 with a donor identifier or a stem cell identifier of the IC tag 18 of the product liquid storing container 20, and the donor identifiers and the stem cell identifiers of the IC tags 18 are matched, a matching OK message and a data storing button are displayed on the display 17. When the donor identifiers and the stem cell identifiers of the IC tags 18 are not matched, the computer 11 displays an error message on the display 17.

Those in charge, after confirming that the culture product liquid 24 stored in the product liquid storing container 20 by the matching OK message is prepared in the process of culturing the dormant stem cell 22 (stem cell) stored in the stem cell storing container 19, store the stem cell storing container 19 and the product liquid storing container 20 in a constant temperature bath (not shown), and maintain the dormant stem cell 22 stored in the stem cell storing container 19 and the culture product liquid 24 stored in the product liquid storing container 20 at room temperature again. Alternatively, the stem cell storing container 19 and the product liquid storing container 20 are allowed to stand indoor for a predetermined period of time, and the dormant stem cell 22 stored in the stem cell storing container 19 and the culture product liquid 24 stored in the product liquid storing container 20 are maintained at room temperature again.

Those in charge prepare a first flat culture container 21 (first culture container), affix an IC tag 18 on the outer peripheral surface of the culture container 21, and then click the data storing button, and using the IC tag reader/writer 12, store on its IC tag 18 the data of the IC tag 18 affixed to the stem cell storing container 19 and the data of the IC tag 18 affixed to the product liquid storing container 20. The computer 11 displays a data storing end message, a dormant stem cell fixation observation button, and a dormant stem cell fixation end button on the display 17.

Those in charge maintain the dormant stem cell 22 and the culture product liquid 24 at room temperature again, and using an injector or a pipette, feed (store) the dormant stem cell 22 into the first flat culture container 21 (first culture container) from the stem cell storing container 19, and using an injector or a pipette, feed (store) the culture liquid 23 into the culture container 21, and using an injector or a pipette, feed (store) the culture product liquid 24 into the culture container 21 from the product liquid storing container 20. The rate of feeding the culture product liquid 24 into the first flat culture container 21 is 5 to 15%, preferably 8 to 12%, more preferably 10%, relative to the total feeding amount (100%) of the culture liquid 23 fed into the culture container 21.

Then, those in charge maintain the first flat culture container 21 into which the dormant stem cell 22, the culture liquid 23, and the culture product liquid 24 are fed at approximately the same temperature as the body temperature of (approx. 37°C), allow it to statically stand for 12 to 24 hours (statically without movement), observe the deformation of the dormant stem cell 22 from the initial plane shape in the culture container 21 at an interval of approx. 1 to 2 hours for the period of 12 to 24 hours, and determine that the dormant stem cell 22 is fixed to the bottom 25 of the culture container 21.

The culture liquid 23 contains a mineral salt solution and an amino acid containing penicillin (approx. 100U/ml), amphotericin (approx. 100ng/ml), streptomycin (approx. 100mkg/ml), L-glutamine (approx. 2 to 4ml), and 20% fetal calf serum. The dormant stem cell 22 fed into the first flat culture container 21 is fixed to the bottom 25 of the culture container 21 as time elapses, cultured by a mixed culture liquid 26 of the culture liquid 23 and the culture product liquid 24, and gradually proliferated (differentiated) on the bottom 25 of the culture container 21 to form a colony.

The culture liquid 23 may be Dulbecco's Modified Eagle's Medium (DMEM), Grasgow Minimum Essential Medium (GMEM), RPMI640, and so on. The culture liquid 23 may contain insulin, transferrin, ethanolamine, selenium, 2-mercaptoethanol, L-alanyl-L-glutamine, sodium pyruvate, L-alanine, L-asparagine, L-aspalathin acid, glycine, L-proline, and L-serine.

The first flat culture container 21 (first culture container) is made of transparent glass or transparent plastics, and a flat container whose plane shape is approximately regular rectangle, having a small capacity and a bottom of a predetermined area. The first flat culture container 21 may be a flat container whose plane shape is circular or elliptical, having a small capacity and a bottom of a predetermined area. The first flat culture container 21 used as a means of fixing a dormant stem cell has a capacity of approx. 20 to 30cc (preferably 25cc), and a bottom-surface area of approx. 25 to 36mm². The culture container 21 has a side 5 to 6mm long.

Those in charge click a dormant stem cell fixation observation button, and set (place) a first flat culture container 21 in a specimen holder 39 of an electron microscope 13. A spacer 42 is positioned between an upper surface 40 of a specimen holder 39 of the electron microscope 13 and a bottom portion 41 of the first flat culture container 21 so as to keep the bottom portion 41 of the culture container 21 raised by the spacer 42, allow the bottom portion 41 of the culture container 21 and the top portion 43 (feed hole 44) of the culture container 21 to stay at higher and lower positions, respectively, and maintain the culture container 21 at a predetermined tilted angle. Also, a spacer 42 may be positioned between the upper surface 40 of the specimen holder 39 of the electron microscope 13 and the top portion 43 of the first flat culture container 21 so as to keep the top portion 43 of the culture container 21 raised by the spacer 42, allow the top portion 43 of the culture container 21 and the bottom portion 41 of the culture container 21 to stay at higher and lower positions, respectively, and maintain the culture container 21 at a predetermined tilted angle. The tilted angle α1 of the first flat culture container 21 in relation to the upper surface 40 of the specimen holder 39 ranges from 2 to 5°, preferably 2 to 3°.

The method for producing a culture product liquid places the first flat culture container 21 in relation to the upper surface 40 of the specimen holder 39 at the tilted angle, thereby allowing the dormant stem cell 22, the culture liquid 23, and the culture product liquid 24 in the culture container 21 to tilt to the side of the top portion 43 of the culture container 21 (or the side of the bottom portion 41), causing the water pressure of the dormant stem cell 22, the culture liquid 23, and the culture product liquid 24 to increase on the side of the top portion 43 of the culture container 21 (or side of the bottom portion 41), allowing the dormant stem cell 22 to concentrate on the side of the bottom portion 43 of the culture container 21 (or side of the bottom portion 41), and thus increasing the activity of the dormant stem cells 22 to readily and immediately fix the dormant stem cell 22 on the bottom 25 of the culture container 21.

The display 17 displays a dormant stem cell fixation observation under-way message and a dormant stem cell fixation end button. The electron microscope 13 images a magnified view of a plane shape of the dormant stem cell 22 fed into the culture container 21 at an interval of approx. 1 to 2 hours, and transmits the magnified view of the plane shape of the dormant stem cell 22 to the computer 11 at an interval of approx. 1 to 2 hours. The interval of imaging or image transmission in the electron microscope 13 can be set at 1 to 2 hours by input devices such as a keyboard 14 and a mouse 15.

The computer 11 stores a magnified view of a plane shape of the dormant stem cell 22 transmitted from the electron microscope 13 and an imaging time in a storage area so as to be associated with a donor identifier and a stem cell identifier. The computer 11 displays the magnified view of the plane shape of the dormant stem cell 22 transmitted from the electron microscope 13 and the imaging time on the display 17. Those in charge confirm (visually recognize) the magnified view of the plane shape of the dormant stem cell 22 displayed on the display 17 at an interval of approx. 1 to 2 hours for 12 to 24 hours, and observe changes in the plane shape of the dormant stem cell 22. Those in charge may directly observe changes in the plane shape of the dormant stem cell 22 from the observation window of the electron microscope 13 at an interval of approx. 1 to 2 hours for 12 to 24 hours.

The initial plane shape of the dormant stem cell 22 (plane shape before being fixed) is an approximately circular shape, and when the plane shape of the dormant stem cell 22 is the approximately circular shape, the dormant stem cell 22 is not fixed to the bottom 25 of the culture container 21 (inner surface of bottom wall), which does not allow the dormant stem cell 22 to start proliferation (differentiation). The plane shape of the dormant stem cell 22 deformed (plane shape after being fixed) is primarily the approximately circular shape before being fixed, and a flat shape when the dormant stem cell 22 elongates (expands) in amorphous shape in one direction (predetermined direction), thereby fixing the dormant stem cell 22 to the bottom 25 of the culture container 21 (inner surface of bottom wall) and allowing the dormant stem cell 22 to start proliferation (differentiation).

Those in charge, according to the observation in the step of fixing a dormant stem cell, as illustrated in FIG. 4, when the magnified view of the plane shape of the dormant stem cell 22 displayed on the display 17 remains observed as an approximately circular shape, determine that the dormant stem cell 22 is not fixed to the bottom 25 of the first flat culture container 21 (inner surface of bottom wall), and continuously observe changes in the plane shape of the dormant stem cell 22 at an interval of approx. 1 to 2 hours. Those in charge, as illustrated in FIG. 5, when the plane shape of the dormant stem cell 11 displayed on the display 17 is deformed from the approximately circular to the primarily approximately circular and amorphous flat shape, determine that the dormant stem cell 11 is fixed to the bottom 25 of the culture container 21.

The use of a large culture container in which the capacity exceeds 30cc and the bottom-surface area exceeds 36mm² when the dormant stem cell 22 is fixed fails to readily fix the dormant cell 22 to the bottom surface of the container and slows down the proliferation of the dormant stem cell 22, but the method for producing a culture product liquid, using the first flat culture container 21 having the capacity and the bottom-surface area, can readily fix the dormant stem cell 22 to the bottom 25 of the culture container 21 and immediately proliferate the dormant stem cell 22 in the culture container 21. The method for producing a culture product liquid allows the first flat culture container 21 to statically stand at approximately the same temperature as the body temperature for a period of 12 to 24 hours, and observes the deformation of the dormant stem cell 22 from the initial plane shape in the culture container 21 at an interval of approx. 1 to 2 hours for the period of 12 to 24 hours, thereby never failing to confirm deformation of the dormant stem cell 22 and properly confirming the fixation of the dormant stem cell 22 to the bottom 25 of the culture container 21.

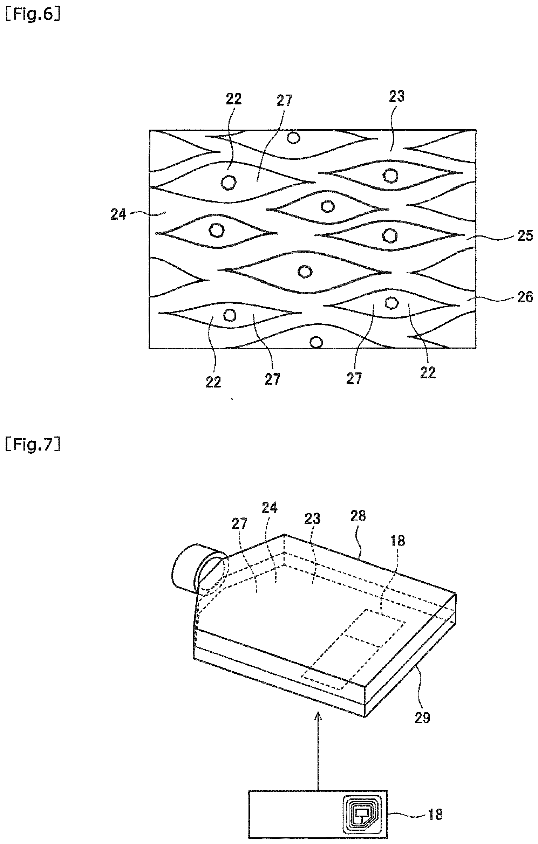

FIG. 6 is a partially enlarged view illustrating another example of a plane shape of a dormant stem cell 22. FIG. 6 is a magnified view of a plane shape of a dormant stem cell 22 imaged with an electron microscope 13. According to the observation in the step of fixing a dormant stem cell step, as illustrated in FIG. 5, the mesenchymal dormant stem cell 22 is deformed from the approximately circular (initial plane shape) to the primarily approximately circular and amorphous flat shape, and a step of confirming the fixation of the dormant stem cell 22 to the bottom 25 of the first flat culture container 21 is followed by a step of culturing a dormant stem cell.

Those in charge such as doctors, nurses, and researchers, after confirming the fixation of the dormant stem cell 22 to the bottom 25 of the first flat culture container 21, click a dormant stem cell fixation end button displayed on the display 17. When the dormant stem cell fixation end button is clicked, the computer 11 displays a dormant stem cell fixation end message, a dormant stem cell culture observation button, and a dormant stem cell culture end button on the display 17.

Those in charge discharge the mixed culture liquid 26 of the culture liquid 23 and the culture product liquid 24 fed into the first flat culture container 21 from the culture container 21 in the step of culturing a dormant stem cell step, and feed (store) a new culture liquid 23 and a new culture product liquid 24 (culture product liquid 24 prepared in the process of culturing a stem cell 30 as an ingredient of the dormant stem cell 22 before storing the dormant stem cell 22) into the culture container 21. The rate of feeding the new culture product liquid 24 into the first flat culture container 21 is 5 to 15%, preferably 8 to 12%, more preferably 10%, relative to the total feeding amount (100%) of the new culture liquid 23 fed into the culture container 21.

Those in charge take the first flat culture container 21 out of the specimen holder 39 of an electron microscope 16, and discharge the mixed culture liquid 26 of the culture liquid 23 and the culture product liquid 24 fed into the culture container 21 from the culture container 21 in the step of fixing a dormant stem cell, using an injector or a pipette, and feed a new culture liquid 23 into a culture container 21, using an injector or a pipette, and feed (store) a new culture product liquid 24 into the culture container 21 from the product liquid storing container 120, using an injector or a pipette. The new culture liquid 23 and the new culture product liquid 24 are the same as those fed into the first flat culture container 21 in the step of fixing a dormant stem cell.

Those in charge allow the first flat culture container 21 to statically stand at approximately the same temperature as the body temperature of (approx. 37°C) for 36 to 48 hours (statically without movement), observe the total area of the dormant stem cell 22 fixed to the bottom 25 of the culture container 21 in relation to the bottom-surface area of the culture container 21 with an electron microscope 13 at an interval of approx. 1 to 2 hours for the period of 36 to 48 hours, and determine whether the total area of the dormant stem cell 22 reaches a target ratio (first target ratio) of the bottom-surface area of the culture container 21 or not. The target ratio of the total area of the dormant stem cell 22 is 70 to 80% (70 to 80% confluent) of the bottom-surface area of the first flat culture container 21.

Those in charge, after feeding a new culture liquid 23 and a new culture product liquid 24 into the first flat culture container 21, click a dormant stem cell culture observation button, and place (set) the culture container 21 in the specimen holder of the electron microscope 13. A spacer 42 is positioned between an upper surface 40 of a specimen holder 39 of the electron microscope 16 and a bottom portion 41 of the first flat culture container 21 so as to keep the bottom portion 41 of the culture container 21 raised by the spacer 42, allow the bottom portion 41 of the culture container 21 and the top portion 43 (feed hole 44) of the culture container 21 to stay at higher and lower positions, respectively, and maintain the culture container 21 at a predetermined tilted angle(see FIG.3). Also, a spacer 42 may be positioned between the upper surface 40 of the specimen holder 39 of the electron microscope 16 and the top portion 43 of the first flat culture container 21 so as to keep the top portion 43 of the culture container 21 raised by the spacer 42, allow the top portion 43 of the culture container 21 and the bottom portion 41 of the culture container 21 to stay at higher and lower positions, respectively, and maintain the culture container 21 at a predetermined tilted angle. The tilted angle α1 of the first flat culture container 21 in relation to the upper surface 40 of the specimen holder 39 ranges from 2 to 5°, preferably 2 to 3°.

The method for producing a culture product liquid, after confirming the fixation of the dormant stem cell 22, discharges a mixed culture liquid 26 in the culture container 21 and feed a new culture liquid 23 and a new culture product liquid 24 into a culture container 21, thereby assuredly encouraging the proliferation of the dormant stem cell 22. The method for producing a culture product liquid places the first flat culture container 21 in relation to the upper surface 40 of the specimen holder 39 at the tilted angle, thereby allowing the dormant stem cell 22, the culture liquid 23, and the culture product liquid 24 in the culture container 21 to tilt to the side of the top portion 43 of the culture container 21 (or the side of the bottom portion 41), causing the water pressure of the dormant stem cell 22, the culture liquid 23, and the culture product liquid 24 to increase on the side of the top portion 43 of the culture container 21 (or side of the bottom portion 41), allowing the dormant stem cell 22 to concentrate on the side of the bottom portion 41 of the culture container 21 (or side of the top portion 43), and thus increasing the activity of the dormant stem cells 22 to readily and immediately fix the dormant stem cell 22 on the bottom 25 of the culture container 21.

The display 17 displays a dormant stem cell culture observation under-way message and a dormant stem cell culture end button. Those in charge, after confirming the fixation of the dormant stem cell 22, discharge a mixed culture liquid 26 of a culture liquid 23 and a culture product liquid 24 from a first flat culture container 21, feed a new culture liquid 23 and a new culture product liquid 24 containing a predetermined metabolite secreted from a single type of stem cell into the culture container 21, and a metabolite of its stem cell 30 itself triggers immediate activation of the dormant stem cell 22 to assuredly encourage the proliferation of the dormant stem cell 22.

The electron microscope 13 images a magnified view of a plane shape of the dormant stem cell 22 fed into the culture container 21 at an interval of approx. 1 to 2 hours, and transmits the magnified view of the plane shape of the dormant stem cell 22 to the computer 11 at an interval of approx. 1 to 2 hours. The interval of imaging or image transmission in the electron microscope 13 can be set at 1 to 2 hours by input devices such as a keyboard 14 and a mouse 15. The computer 11 stores a magnified view of a plane shape of the dormant stem cell 22 transmitted from the electron microscope 13 and an imaging time in a storage area so as to be associated with a donor identifier and a stem cell identifier. The computer 11 displays the magnified view of the plane shape of the dormant stem cell 22 transmitted from the electron microscope 13 and the imaging time on the display 17.

Those in charge confirm (visually recognize) the magnified view of the plane shape of the dormant stem cell 22 displayed on the display 17 at an interval of approx. 1 to 2 hours for 36 to 48 hours, and observe the total area of the dormant stem cell 22 fixed to the bottom 25 of the culture container 21 in relation to the bottom-surface area of the culture container 21, and determine whether the total area of the dormant stem cell 22 reaches a target ratio (first target ratio) (70 to 80% confluent) of the bottom-surface area of the culture container 21 or not. Those in charge may directly observe the total area of the dormant stem cell 22 in relation to the bottom-surface area of the culture container 21 from the observation window of the electron microscope 13 at an interval of approx. 1 to 2 hours for 36 to 48 hours, and determine whether the total area of the dormant stem cell 22 reaches a target ratio (70 to 80% confluent) of the bottom-surface area of the culture container 21 or not.

Those in charge, according to the observation in the step of culturing a dormant stem cell, as illustrated in FIG. 4, when the total area of the dormant stem cell 22 displayed on the display 17 does not reach a target ratio (first target ratio) (70 to 80% confluent) of the bottom-surface area of the first flat culture container 21, continuously observe the total area of the dormant stem cell 22 in relation to the bottom-surface area of the culture container 21 at an interval of approx. 1 to 2 hours. When the total area of the dormant stem cell 22 reaches a target ratio (first target ratio) of the total area of the magnified view displayed on the display 17, it is determined that the total area of the dormant stem cell 22 reaches a target ratio of the bottom-surface area of the first flat culture container 21.

According to the observation in the step of culturing a dormant stem cell, the dormant stem cell 22 is proliferated on the bottom 25 of the first flat culture container 21 (inner surface of bottom wall) to allow the dormant stem cell 22 to form a colony and its plane shape to expand, as illustrated in FIG. 5, when the total area of the dormant stem cell 22 displayed on display 17 reaches a target ratio (first target ratio) (70 to 80% confluent) of the bottom-surface area of the culture container 21, the dormant stem cell 22 is adequately proliferated and activated, and the dormant stem cell 22 is transformed into an activated stem cell 27. When the total area of the dormant stem cell 22 reaches a target ratio of the bottom-surface area of the first flat culture container 21, the activated stem cell 27 is extracted from the culture container 21 and such an activated stem cell 27 extracted is used for each type of disease treatment or regenerative medicine.

Those in charge discharge the mixed culture liquid 26 fed into the first flat culture container 21 from the culture container 21, using an injector or a pipette, and after washing the culture container 21 with PBS, feed a trypsin solution into the culture container 21. When the trypsin solution is fed into the first flat culture container 21, the activated stem cell 27 fixed to the bottom 25 of the culture container 21 peels off the bottom 25 by the trypsin solution, floating up to the surface of the trypsin solution. Those in charge suck the activated stem cell 27 using a pipette, and store the activated stem cell 27 in the pipette. Those in charge can store the activated stem cell 27 cultured in the step of culturing a dormant stem cell in the stem cell storing container 19, place a stem cell storing container 19 that stores the activated stem cell 27 in a refrigerator 14 or a freezer 14, and store the activated stem cell 27 in the refrigerator 14 or the freezer 14.

Those in charge, after confirming that the total area of the dormant stem cell 22 reaches a target ratio (first target ratio) of the bottom-surface area of the first flat culture container 21, click a dormant stem cell culture end button displayed on the display 17. When the dormant stem cell culture end button is clicked, the computer 11 displays an initial screen on the display 17. When the culturing process is finished, a log-out button on the initial screen is clicked. When the log-out button is clicked, the computer 11 logs out of the system.

The method for producing activated stem cells, using a culture product liquid 24 containing a predetermined metabolite secreted from its stem cell 30 prepared in the process of culturing (proliferating) a single type of mesenchymal stem cell 30 before storing a dormant stem cell 22, allows a metabolite of its stem cell 30 itself to trigger immediate activation of the dormant stem cell 22 to encourage the fixation and proliferation of the dormant stem cell 22 to the bottom 25 of the first flat culture container 21 (first culture container), and to maintain the activity of the dormant stem cell 22 and immediately proliferate the dormant stem cell 22.

The method for producing activated stem cells, using a culture product liquid 24 prepared in the process of culturing (proliferating) a single type of mesenchymal stem cell 30 before storing a dormant stem cell 22, fixes the dormant stem cell 22 to the bottom 25 of the first flat culture container 21 (first culture container), and using its culture product liquid 24, proliferates the dormant stem cell 22 until the total area reaches a first target ratio, thereby allowing efficient and assured fixation and proliferation of a single type of mesenchymal dormant stem cell 22, and efficient production of a single type of mesenchymal activated stem cell 27 having adequate activity in required volume.

The method for producing activated stem cells can produce a single type of pure (clear) activated stem cell 27 containing no undesired stem cell by subjecting the mesenchymal dormant stem cell 22 to the steps of fixing a dormant stem cell and culturing a dormant stem cell, and various types of stem cells are not contained, thereby allowing production of an activated stem cell 27 having significant effects in each type of disease treatment and regenerative medicine, and capable of producing an activated stem cell 27 for each type of disease treatment and for regenerating each tissue and organ, both in an appropriate and timely manner.

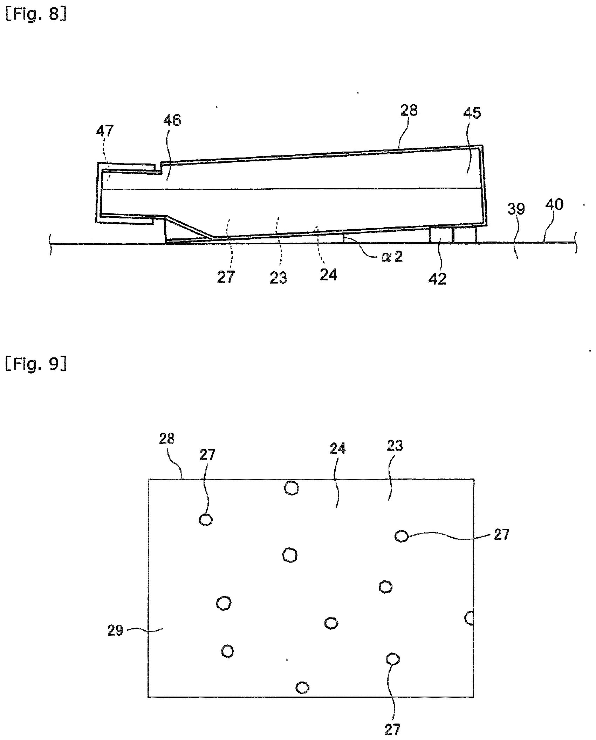

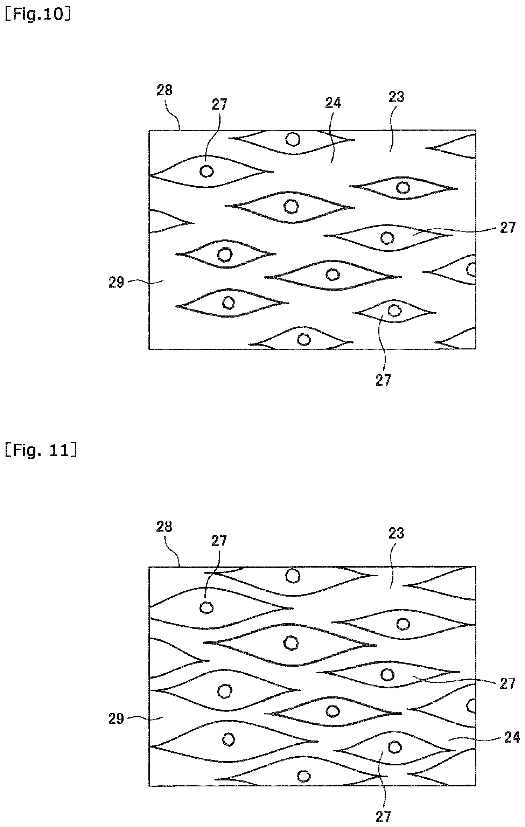

FIG. 7 is an explanatory drawing illustrating one example of a step of fixing an activated stem cell, and FIG. 8 is a side view illustrating a second flat culture container 28. FIG. 9 is a partially enlarged view illustrating one example of a plane shape of an activated stem cell 27, and FIG. 10 is a partially enlarged view illustrating another example of a plane shape of an activated stem cell 27. FIG. 11 is a partially enlarged view illustrating another example of a plane shape of an activated stem cell 27. FIGS. 9 to 11 illustrate magnified views of a plane shape of an activated stem cell 27 imaged with an electron microscope 13.

Those in charge, after culturing a dormant stem cell 22 (activated stem cell 27) by the step of culturing a dormant stem cell, in order to continuously culture an activated stem cell 27, click a dormant stem cell culture end button, followed by clicking an activated stem cell culture button on the initial screen displayed on the display 17. When the activated stem cell culture button is clicked, the computer 11 displays a data storing button on the display 17.

Those in charge such as doctors, nurses, and researchers prepare a second flat culture container 28 (second culture container), affix an IC tag 18 on the outer peripheral surface of the culture container 28, and then click the data storing button, and using an IC tag reader/writer 12, store in its IC tag 18 the data of the IC tag 18 affixed to the first flat culture container 21 (first culture container). The computer 11 displays a data storing end message, an activated stem cell fixation observation button, and an activated stem cell fixation end button on the display 17.

Those in charge feed (store) the activated stem cell 27 into the second flat culture container 28 from the first flat culture container 21 (first culture container), using an injector or a pipette, feed (store) the culture liquid 23 into the second flat culture container 28, using an injector or a pipette, and feed (store) the culture product liquid 24 into the second flat culture container 28 from the product liquid storing container 20, using an injector or a pipette. The rate of feeding the culture product liquid 24 into the second flat culture container 28 is 5 to 15%, preferably 8 to 12%, more preferably 10%, relative to the total feeding amount (100%) of the culture liquid 23 fed into the second flat culture container 28. The culture liquid 23 and the culture product liquid 24 are the same as those fed into the first flat culture container 21.

Then, those in charge maintain the second flat culture container 28 into which the activated stem cell 27, the culture liquid 23, and the culture product liquid 24 are fed at approximately the same temperature as the body temperature of (approx. 37°C), allow it to statically stand for 36 to 48 hours (statically without movement), observe the deformation of the activated stem cell 27 from the initial plane shape in the culture container 28 with an electron microscope 13 at an interval of approx. 1 to 2 hours for the period of 12 to 24 hours, and determine that the activated stem cell 27 is fixed to the bottom 29 of the culture container 28.

The second flat culture container 28 (second culture container) is made of transparent glass or transparent plastics, and a flat container whose plane shape is approximately regular rectangle, having a small capacity and a bottom of a predetermined area, the area of a bottom 29 being approx. twice the first flat culture container 21 (first culture container). The second flat culture container 28 may be a flat container whose plane shape is circular or elliptical, having a small capacity and a bottom of a predetermined area. The second flat culture container 28 used as a means of fixing an activated stem cell has a capacity of approx. 40 to 60cc (preferably 50cc), and a bottom-surface area of approx. 50 to 72mm². The culture container 28 has a side 7 to 8.5mm long.

Those in charge click an activated stem cell fixation observation button, and place (set) the second flat culture container 28 in a specimen holder 39 of the electron microscope 13. A spacer 42 is positioned between an upper surface 40 of the specimen holder 39 of the electron microscope 13 and a bottom portion 45 of the second flat culture container 28 so as to keep the bottom portion 45 of the culture container 28 raised by the spacer 42, allow the bottom portion 45 of the culture container 28 and the top portion 46 (feed hole 47) of the culture container 28 to stay at higher and lower positions, respectively, and maintain the culture container 28 at a predetermined tilted angle. Also, a spacer 42 may be positioned between the upper surface 40 of the specimen holder 39 of the electron microscope 13 and the top portion 46 of the second flat culture container 28 so as to keep the top portion 46 of the culture container 28 raised by the spacer 42, allow the top portion 46 of the culture container 28 and the bottom portion 45 of the culture container 28 to stay at higher and lower positions, respectively, and maintain the culture container 28 at a predetermined tilted angle. The tilted angle α2 of the second flat culture container 28 in relation to the upper surface 40 of the specimen holder 39 ranges from 2 to 5°, preferably 2 to 3°.

The method for producing a culture product liquid places the second flat culture container 28 in relation to the upper surface 40 of the specimen holder 39 at the tilted angle, thereby allowing the activated stem cell 27, the culture liquid 23, and the culture product liquid 24 in the culture container 28 to tilt to the side of the top portion 46 of the culture container 28 (or the side of the bottom portion 45), causing the water pressure of the activated stem cell 27, the culture liquid 23, and the culture product liquid 24 to increase on the side of the top portion 46 of the culture container 28 (or side of the bottom portion 45), allowing the activated stem cell 27 to concentrate on the side of the top portion 46 of the culture container 28 (or side of the bottom portion 45), and thus increasing the activity of the activated stem cells 27 to readily and immediately fix the activated stem cell 27 on the bottom 29 of the culture container 28.

The display 17 displays an activated stem cell fixation observation under-way message and an activated stem cell fixation end button. The electron microscope 13 images a magnified view of a plane shape of the activated stem cell 27 fed into the second flat culture container 28 at an interval of approx. 1 to 2 hours, and transmits the magnified view of the plane shape of the activated stem cell 27 to the computer 11 at an interval of approx. 1 to 2 hours. The interval of imaging or image transmission in the electron microscope 13 can be set at 1 to 2 hours by input devices such as a keyboard 14 and a mouse 15.

The computer 11 stores a magnified view of a plane shape of the activated stem cell 27 transmitted from the electron microscope 13 and an imaging time in a storage area so as to be associated with a donor identifier and a stem cell identifier. The computer 11 displays the magnified view of the plane shape of the activated stem cell 27 transmitted from the electron microscope 13 and the imaging time on the display 17. Those in charge confirm (visually recognize) the magnified view of the plane shape of the activated stem cell 27 displayed on the display 17 at an interval of approx. 1 to 2 hours for 12 to 24 hours, and observe changes in the plane shape of the activated stem cell 27. Those in charge may directly observe changes in the plane shape of the activated stem cell 27 from the observation window of the electron microscope 13 at an interval of approx. 1 to 2 hours for 12 to 24 hours.

The initial plane shape of the activated stem cell 27 (plane shape before being fixed) is an approximately circular shape, and when the plane shape of the activated stem cell 27 is the approximately circular shape, the activated stem cell 27 is not fixed to the bottom 29 of the second flat culture container 28 (inner surface of bottom wall), which does not allow the activated stem cell 27 to start proliferation (differentiation). The plane shape of the activated stem cell 27 deformed (plane shape after being fixed) is primarily the approximately circular shape before being fixed, and a flat shape when the activated stem cell 27 elongates (expands) in amorphous shape in one direction (predetermined direction), thereby fixing the activated stem cell 27 to the bottom 28 of the second flat culture container 28 (inner surface of bottom wall) and allowing the activated stem cell 27 to start proliferation (activation).

Those in charge, according to the observation in the step of fixing an activated stem cell, as illustrated in FIG. 8, when the magnified view of the plane shape of the activated stem cell 27 displayed on the display 17 remains observed as an approximately circular shape, determine that the activated stem cell 27 is not fixed to the bottom 29 of the second flat culture container 28 (inner surface of bottom wall), and continuously observe changes in the plane shape of the activated stem cell 27 at an interval of approx. 1 to 2 hours. Those in charge, as illustrated in FIG. 9, when the plane shape of the activated stem cell 27 displayed on the display 17 is deformed from the approximately circular to the primarily approximately circular and amorphous flat shape, determine that the activated stem cell 27 is fixed to the bottom 29 of the second flat culture container 28.

The use of a large culture container in which the capacity exceeds 60cc and the bottom-surface area exceeds 72mm² when the activated stem cell 27 is fixed fails to readily fix the activated cell 27 to the bottom surface of the container and slows down the proliferation of the activated stem cell 27, but the method for producing a culture product liquid, using a second flat culture container 28 having the capacity and the bottom-surface area, can readily fix the activated stem cell 27 to the bottom 29 of the culture container 28 and immediately proliferate the activated stem cell 27 in the culture container 28. The method for producing a culture product liquid allows the second flat culture container 28 to statically stand at approximately the same temperature as the body temperature for a period of 36 to 48 hours, and observes the deformation of the activated stem cell 27 from the initial plane shape in the culture container 28 at an interval of approx. 1 to 2 hours for the period of 12 to 24 hours, thereby never failing to confirm deformation of the activated stem cell 27 and properly confirming the fixation of the activated stem cell 27 to the bottom 29 of the culture container 28.

According to the observation in the step of fixing an activated stem cell step, as illustrated in FIG. 9, the mesenchymal activated stem cell 27 is deformed from the approximately circular (initial plane shape) to the primarily approximately circular and amorphous flat shape, and a step of confirming the fixation of the activated stem cell 27 to the bottom 29 of the second flat culture container 28 is followed by a step of culturing an activated stem cell. Those in charge such as doctors, nurses, and researchers, after confirming the fixation of the activated stem cell 27 to the bottom 29 of the second flat culture container 28, click an activated stem cell fixation end button displayed on the display 17. When the activated stem cell fixation end button is clicked, the computer 11 displays an activated stem cell fixation end message, an activated stem cell culture observation button, and an activated stem cell culture end button on the display 17.

Those in charge discharge a mixed culture liquid 26 of the culture liquid 23 and the culture product liquid 24 fed into the second flat culture container 28 from the culture container 28 in the step of culturing an activated stem cell step from, and feed (store) a new culture liquid 23 and a new culture product liquid 24 (culture product liquid 24 prepared in the process of culturing a stem cell 30 as an ingredient of the dormant stem cell 22 before storing the dormant stem cell 22) into the culture container 28. The rate of feeding the new culture product liquid 24 into the second flat culture container 28 is 5 to 15%, preferably 8 to 12%, more preferably 10%, relative to the total feeding amount (100%) of the new culture liquid 23 fed into the culture container 28.

Those in charge take the second flat culture container 28 out of a specimen holder of an electron microscope 16, and discharge a mixed culture liquid 26 of the culture liquid 23 and the culture product liquid 24 fed into the culture container 28 from the culture container 28 in the step of fixing an activated stem cell, using an injector or a pipette, and feed (store) a new culture liquid 23 into a culture container 28, using an injector or a pipette, and feed (store) a new culture product liquid 24 into the culture container 28 from the product liquid storing container 20, using an injector or a pipette. The new culture liquid 23 and the new culture product liquid 24 are the same as those fed into the first second culture container 28 in the step of fixing an activated stem cell.

Those in charge maintain the second flat culture container 28 at approximately the same temperature as the body temperature of (approx. 37°C), allow it to statically stand for 36 to 48 hours (statically without movement), observe the total area of the activated stem cell 27 fixed to the bottom 29 of the culture container 28 in relation to the bottom-surface area of the culture container 28 with an electron microscope 13 at an interval of approx. 1 to 2 hours for the period of 36 to 48 hours, and determine whether the total area of the activated stem cell 27 reaches a target ratio (second target ratio) of the bottom-surface area of the culture container 28 or not. The target ratio of the total area of the activated stem cell 27 is 88 to 92% (88 to 92% confluent) of the bottom-surface area of the first flat culture container 28.

Those in charge, after feeding a new culture liquid 23 and a new culture product liquid 24 into the second flat culture container 28, click an activated stem cell culture observation button, and place (set) the culture container 28 in a specimen holder of the electron microscope 13. A spacer 42 is positioned between an upper surface 40 of a specimen holder 39 of the electron microscope 16 and a bottom portion 45 of the first flat culture container 28 so as to keep the bottom portion 45 of the culture container 28 raised by the spacer 42, allow the bottom portion 45 of the culture container 28 and the top portion 46 (feed hole 47) of the culture container 28 to stay at higher and lower positions, respectively, and maintain the culture container 28 at a predetermined tilted angle (see FIG.8). Also, a spacer 42 may be positioned between the upper surface 40 of the specimen holder 39 of the electron microscope 16 and the top portion 46 of the first flat culture container 28 so as to keep the top portion 46 of the culture container 28 raised by the spacer 42, allow the top portion 46 of the culture container 28 and the bottom portion 45 of the culture container 28 to stay at higher and lower positions, respectively, and maintain the culture container 28 at a predetermined tilted angle. The tilted angle α2 of the second flat culture container 28 in relation to the upper surface 40 of the specimen holder 39 ranges from 2 to 5°, preferably 2 to 3°.

The method for producing a culture product liquid, after confirming the fixation of the activated stem cell 27, discharges a mixed culture liquid 26 in the culture container 28 and feed a new culture liquid 23 and a new culture product liquid 24 into a culture container 28, thereby assuredly encouraging the proliferation of the activated stem cell 27. The method for producing a culture product liquid places a second flat culture container 28 in relation to the upper surface 40 of the specimen holder 39 at the tilted angle, thereby allowing the activated stem cell 27, the culture liquid 23, and the culture product liquid 24 in the culture container 28 to tilt to the side of the top portion 46 of the culture container 28 (or the side of the bottom portion 45), causing the water pressure of the activated stem cell 27, the culture liquid 23, and the culture product liquid 24 to increase on the side of the top portion 46 of the culture container 28 (or side of the bottom portion 45), allowing the activated stem cell 27 to concentrate on the side of the bottom portion 45 of the culture container 28 (or side of the top portion 46), and thus increasing (differentiation) the activity of the activated stem cells 27 to readily and immediately fix the activated stem cell 27 on the bottom 29 of the culture container 27.

The display 17 displays an activated stem cell culture observation under-way message and an activated stem cell culture end button. Those in charge, after confirming the fixation of the activated stem cell 27, discharge the mixed culture liquid 26 of the culture liquid 23 and the culture product liquid 24 from the second flat culture container 28, feed a new culture liquid 23 and a new culture product liquid 24 containing a predetermined metabolite secreted from a single type of stem cell 30 into the second flat culture container 28, and a metabolite of its stem cell 30 itself triggers immediate activation of the activated stem cell 27 to assuredly encourage the proliferation of the activated stem cell 27.

The electron microscope 13 images a magnified view of a plane shape of the activated stem cell 27 in the second flat culture container 28 at an interval of approx. 1 to 2 hours, and transmits the magnified view of the plane shape of the activated stem cell 27 to the computer 11 at an interval of approx. 1 to 2 hours. The interval of imaging or image transmission in the electron microscope 13 can be set at 1 to 2 hours by input devices such as a keyboard 14 and a mouse 15. The computer 11 stores the magnified view of the plane shape of the activated stem cell 27 transmitted from the electron microscope 13 and an imaging time in a storage area so as to be associated with a donor identifier and a stem cell identifier. The computer 11 displays the magnified view of the plane shape of the activated stem cell 27 transmitted from the electron microscope 13 and the imaging time on the display 17.

Those in charge confirm (visually recognize) the magnified view of the plane shape of the activated stem cell 27 displayed on the display 17 at an interval of approx. 1 to 2 hours for 36 to 48 hours, and observe the total area of the activated stem cell 27 fixed to the bottom 29 of the second flat culture container 28 in relation to the bottom-surface area of the culture container 28, and determine whether the total area of the activated stem cell 27 reaches a target ratio (second target ratio) (88 to 92% confluent) of the bottom-surface area of the culture container 28 or not. Those in charge may directly observe the total area of the activated stem cell 27 in relation to the bottom-surface area of the second flat culture container 28 from the observation window of the electron microscope 13 at an interval of approx. 1 to 2 hours for 36 to 48 hours, and determine whether the total area of the activated stem cell 27 reaches a target ratio (88 to 92% confluent) of the bottom-surface area of the culture container 28 or not.

Those in charge, according to the observation in the step of culturing an activated stem cell, as illustrated in FIG. 9, when the total area of the activated stem cell 27 displayed on the display 17 does not reach a target ratio (second target ratio) (88 to 92% confluent) of the bottom-surface area of the second flat culture container 28, continuously observe the total area of the activated stem cell 27 in relation to the bottom-surface area of the culture container 28 at an interval of approx. 1 to 2 hours. When the total area of the activated stem cell 27 reaches a target ratio (second target ratio) of the total area of the magnified view displayed on the display 17, it is determined that the total area of the activated stem cell 27 reaches a target ratio of the bottom-surface area of the second flat culture container 28.

According to the observation in the step of culturing an activated stem cell, the activated stem cell 27 is proliferated on the bottom 29 of the second flat culture container 28 (inner surface of bottom wall) to allow the activated stem cell 27 to form a colony and its plane shape to expand, and as illustrated in FIG. 10, when the total area of the activated stem cell 27 displayed on display 17 reaches a target ratio (second target ratio) of the bottom-surface area of the culture container 28 (88 to 92% confluent), the activated stem cell 27 is further proliferated and activated. When the total area of the activated stem cell 27 reaches a target ratio of the bottom-surface area of the second flat culture container 28, the activated stem cell 27 is extracted from the culture container 28 and such an activated stem cell 27 extracted is used for each type of disease treatment or regenerative medicine.

Those in charge discharge the mixed culture liquid 26 fed into the second flat culture container 28 from the culture container 28, using an injector or a pipette, and after washing the culture container 28 with PBS, feed a trypsin solution into the culture container 28. When the trypsin solution is fed into the second flat culture container 28, the activated stem cell 27 fixed to the bottom 29 of the culture container 28 peels off the bottom 29 by the trypsin solution, floating up to the surface of the trypsin solution. Those in charge suck the activated stem cell 27 using a pipette, and store the activated stem cell 27 in the pipette. Those in charge can store the activated stem cell 27 cultured in the step of culturing an activated stem cell in the stem cell storing container 19, place the stem cell storing container 19 that stores the activated stem cell 27 in a refrigerator 14 or a freezer 14, and store the activated stem cell 27 in the refrigerator 14 or freezer 14.

Those in charge, after confirming that the total area of the activated stem cell 27 reaches a target ratio (second target ratio) of the bottom-surface area of the second flat culture container 28, click an activated stem cell culture end button displayed on the display 17. When the activated stem cell culture end button is clicked, the computer 11 displays an initial screen on the display 17. When the culturing process is finished, a log-out button on the initial screen is clicked. When the log-out button is clicked, the computer 11 logs out of the system 10.

The method for producing activated stem cells, using a culture product liquid 24 containing a predetermined metabolite secreted from its stem cell 30 prepared in the process of culturing (proliferating) a single type of mesenchymal stem cell 30 before storing an activated stem cell 27(dormant stem cell 22), allows a metabolite of its stem cell 30 itself to trigger immediate activation of the activated stem cell 27 to encourage the fixation and proliferation of the activated stem cell 27 to the bottom 29 of the second flat culture container 28 (second culture container), and to maintain the activity of the activated stem cells 27 and immediately proliferate the activated stem cell 27.

The method for producing activated stem cells, using a culture product liquid 24 prepared in the process of culturing (proliferating) a single type of mesenchymal stem cell 30 before storing an activated stem cell 27 (dormant stem cell 22), fixes the activated stem cell 27 to a bottom 29 of a second flat culture container 28 (second culture container), and using its culture product liquid 24, proliferates the activated stem cell 27 until the total area reaches a second target ratio, thereby allowing efficient and assured fixation and proliferation of a single type of mesenchymal activated stem cell 27, and efficient production of a single type of mesenchymal activated stem cell 27 having adequate activity in required volume.

The method for producing activated stem cells can produce a single type of pure (clear) activated stem cell containing no undesired stem cell by subjecting the mesenchymal activated stem cell 27 to the steps of fixing an activated stem cell and culturing an activated stem cell, and various types of stem cells are not contained, thereby allowing production of an activated stem cell 27 having significant effects in each type of disease treatment and regenerative medicine, and production of an activated stem cell 27 for each type of disease treatment and for regenerating each tissue and organ, both in an appropriate and timely manner.

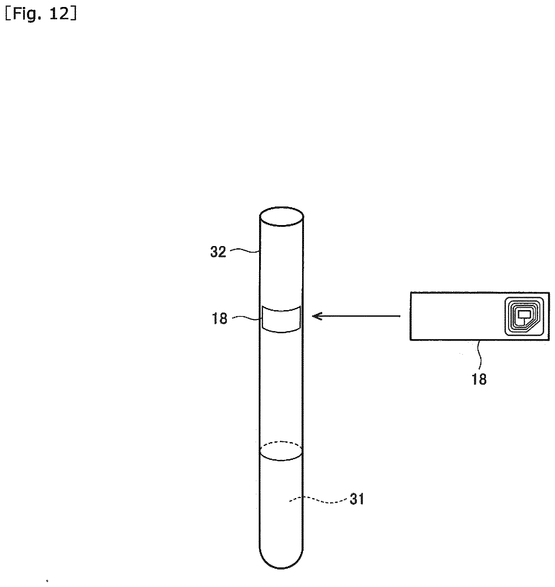

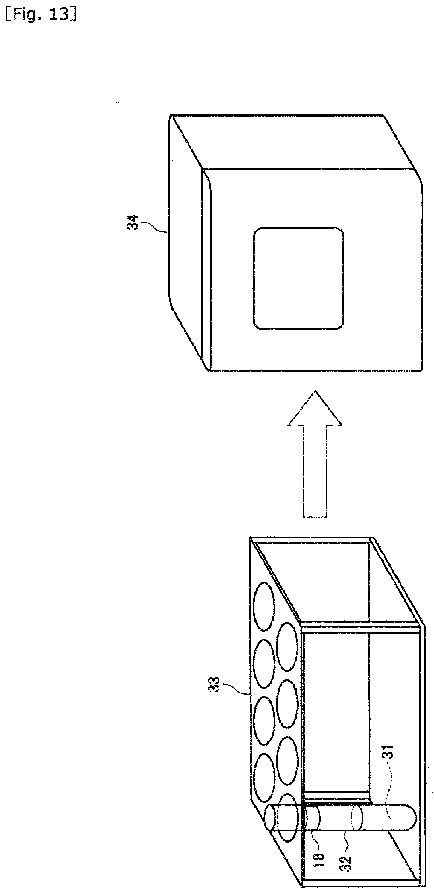

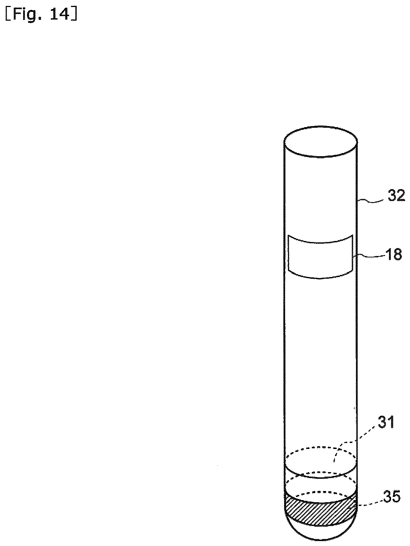

FIG. 12 is a perspective view illustrating a glass test tube 32 used in a step of first fixing a stem cell, and FIG. 13 is an explanatory drawing illustrating a step of first fixing a stem cell following the step in FIG. 12. FIG. 14 is an explanatory drawing illustrating a step of first fixing a stem cell following the step in FIG. 13. With reference to FIGS. 12 to 22, a method for producing a single type of mesenchymal stem cell 30 as an ingredient of a dormant stem cell 22 before storing the dormant stem cell 22 will be described.

A single type of stem cell 30, using a raw bone marrow liquid 31 collected from a plurality of donors (humans), is prepared by an activated stem cell culture system 10, via a step of first fixing a stem cell, a step of first culturing a stem cell, a step of second fixing a stem cell, and a step of second culturing a stem cell. The system 10 is configured to culture a single and specific type of mesenchymal stem cell 38 (first stem cell) out of a plurality of types of mesenchymal stem cells contained in the raw bone marrow liquid 31.

The step of first fixing a stem cell first separates in layers a raw bone marrow liquid 31 collected from a donor. In the step of collecting a bone marrow liquid, 2 to 3cc (2 to 3ml) of the raw bone marrow liquid 31 is collected from the breastbone or ilium (pelvis) of the donor. The raw bone marrow liquid 31, after local anesthesia is used on the donor, is collected by "bone marrow puncture" (a.k.a. "MARK", originating in a German word, "Knochenmark", meaning bone marrow) for puncturing a bone marrow and sucking a bone marrow liquid (bone marrow blood). Those in charge such as doctors, nurses, and researchers, upon collection of the raw bone marrow liquid 31, activate the system 10 on the computer 11, and click a stem cell culture button on an initial screen. The display 17 displays a data acquisition button and a log-out button.

Those in charge click the data acquisition button, and then input donor data and stem cell data into the computer 11, using input devices such as a keyboard 14 and a mouse 15. The computer 11 generates a unique donor identifier and a stem cell identifier for identifying each donor each time donor data and stem cell data are input (the raw bone marrow liquid is collected from the donor), and stores the donor data and the stem cell data in a storage area so as to be associated with a donor identifier and a stem cell identifier. The display 17 displays a donor data acquisition message, a data storing button, and a log-out button.

2 to 3cc of a raw bone marrow liquid 31 collected from a donor, as illustrated in FIG. 12, is fed (stored) into a vertically elongating glass test tube 32 (separation container). 2 to 3cc of the raw bone marrow liquid 31 contains 0.5 to 1ml (approx. 5×10⁷ (cells/ml)) of a plurality of types of mesenchymal stem cells.

On the outer peripheral surface of the glass test tube 32 for feeding the raw bone marrow liquid 31 is affixed an IC tag 18. Those in charge, using an IC tag reader/writer 12, write donor data and stem cell data on the IC tag 18 of the glass test tube 32. When the donor data and the stem cell data are written on the IC tag 18, the computer 11 displays a data storing end message, a bone marrow liquid separation button, and a log-out button on the display 17.

The glass test tube 32 in which a raw bone marrow liquid 31 is fed, as illustrated in FIG. 13, is set in a test tube rack 33, and stored in a constant temperature bath 34 together with the test tube rack 33. Those in charge, after clicking a bone marrow liquid separation button displayed on the display 17, feed the raw bone marrow liquid 31 into the glass test tube 32 from the injector, and insert (set) into the test tube rack 33 the glass test tube 32 into which the raw bone marrow liquid 31 is fed. The display 17 displays a bone marrow liquid separation under-way message and a bone marrow liquid separation end button.

Those in charge store the test tube rack 33 in the constant temperature bath 34, and allow the glass test tube 32 into which the raw bone marrow liquid 31 is fed to statically stand in the constant temperature bath 34 for a predetermined period of time (approx. 2 hours) (statically without movement). The temperature in the constant temperature bath 34 is maintained at approximately the same temperature as the body temperature of approx. 37°C. By allowing the glass test tube 32 to statistically stand in the constant temperature bath 34 for a predetermined period of time (approx. 2 hours), as illustrated in FIG. 14, the raw bone marrow liquid 31 fed into the test tube 32 is vertically separated in layers in the test tube 32 (separated in 3 layers in FIG. 14).

A step of separating in layers the bone marrow liquid 31 is followed by a step of extracting a bone marrow liquid. In the step of extracting a bone marrow liquid, an intermediate-layer bone marrow liquid 35 is extracted from the bone marrow liquid 31 separated in layers. Those in charge take the test tube rack 33 out of the constant temperature bath 34, pull the glass test tube 32 out of the test tube rack 33, confirm the separation of the raw bone marrow liquid 31 in layers, and extract an intermediate-layer bone marrow liquid 35 present in a specific layer of the raw bone marrow liquid 31 separated in layers.

Those in charge suck the intermediate-layer bone marrow liquid 35, 3 to 4mm thick, positioned in an intermediate layer out of the raw bone marrow liquid 31 separated in layers, using an injector or a pipette. Those in charge, after vertically separating in layers the raw bone marrow liquid 31 containing various types of mesenchymal stem cells, extract a specific intermediate-layer bone marrow liquid 35 from the raw bone marrow liquid 31, thereby allowing removal of undesired mesenchymal stem cell contained in the raw bone marrow liquid 31.

After the intermediate-layer bone marrow liquid 35 is extracted, a bone marrow liquid separation end button is clicked. The display 17 displays a bone marrow liquid separation end message and a data storing button. Those in charge prepare a third flat culture container (third culture container) (not shown), affix an IC tag on the outer peripheral surface of the culture container, then click a data storing button, and using an IC tag reader/writer 12, store donor data and stem cell data of the IC tag 18 affixed to the glass test tube 32. The computer 11 displays a data storing end message, a stem cell first fixing observation button, and a stem cell first fixing end button on the display 17.

Those in charge, after extracting a specific intermediate-layer bone marrow liquid 35 positioned in an intermediate layer out of the raw bone marrow liquid 31, feed (store) the intermediate-layer bone marrow liquid 35 and the culture liquid 23 into the third flat culture container (third culture container), using an injector or a pipette, maintain the culture container at approximately the same temperature as the body temperature of approx. 37°C, allow the container to statistically stand for 12 to 24 hours (statically without movement), observe the deformation of the mesenchymal stem cell 38 (first stem cell) contained in the intermediate-layer bone marrow liquid 35 in the culture container from the initial plane shape at an interval of approx. 1 to 2 hours for the period of 12 to 24 hours, and determine whether the stem cell 36 is fixed to the bottom of the culture container or not.

A third flat culture container used in a step of first fixing a stem cell and a step of first culturing a stem cell is the same (in shape and size) as the first flat culture container 21 (first culture container) used in the step of fixing a dormant stem cell and the step of culturing a dormant stem cell (see FIG. 2). The third flat culture container has a capacity of approx. 20 to 30cc (preferably 25cc), and a bottom-surface area of approx. 25 to 36mm2. The third flat culture container has a side 5 to 6mm long.

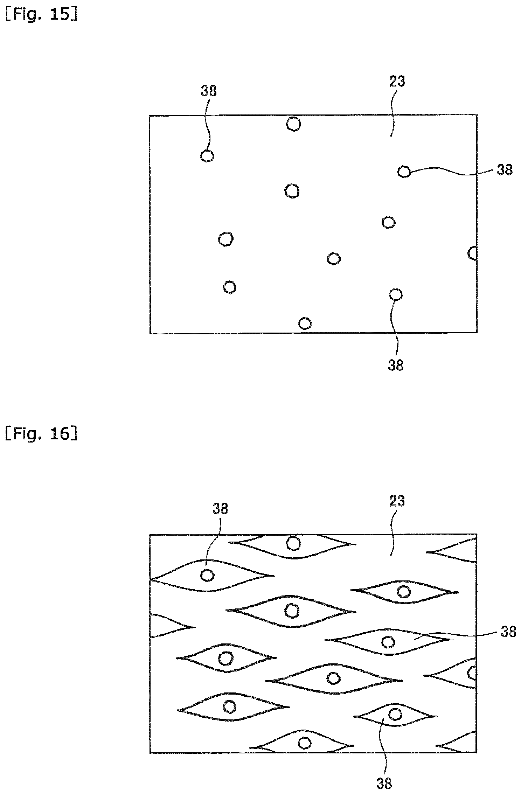

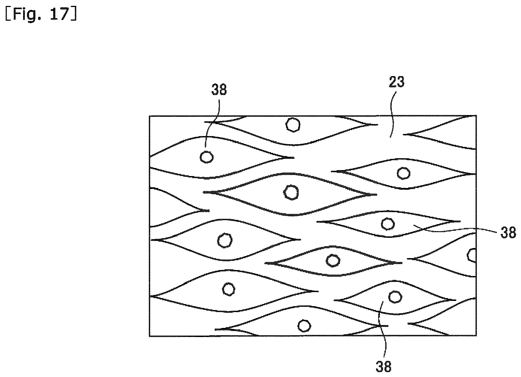

FIG. 15 is a partially enlarged view illustrating one example of a plane shape of a stem cell 38. FIG. 16 is a partially enlarged view illustrating another example of a plane shape of a stem cell 38, and FIG. 17 is a partially enlarged view illustrating another example of a plane shape of a stem cell 38. FIGS. 15 to 17 illustrate magnified views of a plane shape of a stem cell 38 imaged with an electron microscope 13.

Those in charge click a first fixing observation button, and set (place) a third flat culture container in a specimen holder 39 of an electron microscope 13. A spacer 42 is positioned between an upper surface 40 of the specimen holder 39 of the electron microscope 13 and a bottom portion of the third flat culture container so as to keep the bottom portion of the third flat culture container raised by the spacer 42, allow the bottom portion of the third flat culture container and the top portion (feed hole) of the third flat culture container to stay at higher and lower positions, respectively, and maintain the third flat culture container at a predetermined tilted angle (see FIG. 3). Also, a spacer 42 may be positioned between the upper surface 40 of the specimen holder 39 of the electron microscope 13 and the top portion of the third flat culture container so as to keep the top portion of the third flat culture container raised by the spacer 42, allow the top portion of the third flat culture container and the bottom portion of the third flat culture container to stay at higher and lower positions, respectively, and maintain the third flat culture container at a predetermined tilted angle. The tilted angle α1 of the third flat culture container in relation to the upper surface 40 of the specimen holder 39 ranges from 2 to 5°, preferably 2 to 3°.

The method for producing a culture product liquid places the third flat culture container in relation to the upper surface 40 of the specimen holder 39 at the tilted angle, thereby allowing the stem cell 38 and the culture liquid 23 in the third flat culture container to tilt to the side of the top portion of the third flat culture container (or the side of the bottom portion), causing the water pressure of the stem cell 38 and the culture liquid 23 to increase on the side of the top portion of the third flat culture container (or side of the bottom portion), allowing the stem cell 38 to concentrate on the side of the top portion (or side of the bottom portion) of the third flat culture container, and thus increasing the activity of the stem cells 38 to readily and immediately fix the stem cell 38 on the bottom 25 of the third flat culture container.

The display 17 displays a stem cell first fixing observation under-way message and a stem cell first fixing end button. The electron microscope 13 images a magnified view of a plane shape of the stem cell 38 contained in the intermediate-layer bone marrow liquid 35 fed into the third flat culture container at an interval of approx. 1 to 2 hours, and transmits the magnified view of the plane shape of the stem cell 38 to the computer 11 at an interval of approx. 1 to 2 hours.

The interval of imaging or image transmission in the electron microscope 13 can be set at 1 to 2 hours by input devices such as a keyboard 14 and a mouse 15. The mesenchymal stem cell 38 (first stem cell) fed into the third flat culture container is fixed to the bottom of the culture container as time elapses, cultured by a culture liquid, and gradually proliferated (differentiated) on the bottom of the culture container to form a colony.

The computer 11 stores a magnified view of a plane shape of the mesenchymal stem cell 38 (first stem cell) transmitted from the electron microscope 13 and an imaging time in a storage area so as to be associated with a donor identifier and a stem cell identifier. The computer 11 displays the magnified view of the plane shape of the stem cell 38 transmitted from the electron microscope 13 and the imaging time on the display 17. Those in charge confirm (visually recognize) the magnified view of the plane shape of the stem cell 38 displayed on the display 17 at an interval of approx. 1 to 2 hours for 12 to 24 hours, and observe changes in the plane shape of the stem cell 38. Those in charge may directly observe changes in the plane shape of the stem cell 38 from the observation window of the electron microscope 13 at an interval of approx. 1 to 2 hours for 12 to 24 hours.

The initial plane shape of the stem cell 38 (first stem cell) is an approximately circular shape, and when the plane shape of the stem cell 38 is the approximately circular shape, the stem cell 38 is not fixed to the bottom of the third flat culture container (inner surface of bottom wall), which does not allow the stem cell 38 to start proliferation (differentiation). The plane shape of the stem cell 38 (first stem cell) deformed (plane shape after being fixed) is primarily the approximately circular shape before being fixed, and a flat shape when the stem cell 38 elongates (expands) in amorphous shape in one direction (predetermined direction), thereby fixing the stem cell 38 to the bottom of the culture container 38 (inner surface of bottom wall) and allowing the stem cell 38 to start proliferation (activation).

Those in charge, according to the observation in the step of first fixing a stem cell, as illustrated in FIG. 15, when the magnified view of the plane shape of the stem cell 38 (first stem cell) displayed on the display 17 remains observed as an approximately circular shape, determine that the stem cell 38 is not fixed to the bottom of the third flat culture container (inner surface of bottom wall), and continuously observe changes in the plane shape of the stem cell 38 at an interval of approx. 1 to 2 hours. Those in charge, as illustrated in FIG. 16, when the plane shape of the stem cell 38 (first stem cell) displayed on the display 17 is deformed from the approximately circular to the primarily approximately circular and amorphous flat shape, determine that the stem cell 38 is fixed to the bottom of the culture container.

The use of a large culture container in which the capacity exceeds 30cc and the bottom-surface area exceeds 36mm² when the stem cell 38 (first stem cell) is fixed fails to readily fix the stem cell 38 to the bottom surface of the container and slows down the proliferation of the stem cell 38, but the method for producing a culture product liquid, using the third flat culture container having the capacity and the bottom-surface area as the first flat culture container 21, can readily fix the stem cell 38 to the bottom of the culture container and immediately proliferate the stem cell 38 in the culture container. The method for producing a culture product liquid allows the third flat culture container to statically stand at approximately the same temperature as the body temperature for a period of 12 to 24 hours, and observes the deformation of the stem cell 38 (first stem cell) from the initial plane shape in the culture container at an interval of approx. 1 to 2 hours for the period of 12 to 24 hours, thereby never failing to confirm deformation of the stem cell 38 and properly confirming the fixation of the stem cell 38 to the bottom of the third flat culture container.

According to the observation in the step of first fixing a stem cell step, the mesenchymal stem cell 38 (first stem cell) is deformed from the approximately circular (initial plane shape) to the primarily approximately circular and amorphous flat shape, and a step of confirming the fixation of the stem cell 38 to the bottom of the third flat culture container is followed by a step of first culturing a stem cell. Those in charge such as doctors, nurses, and researchers, after confirming the fixation of the stem cell 38 to the bottom of the third flat culture container, click a first fixing end button displayed on the display 17. When the first fixing end button is clicked, the computer 11 displays a stem cell first fixing end message, a stem cell first culture observation button, and a stem cell first culture end button on the display 17.

Those in charge discharge the culture liquid 23 fed into the third flat culture container from the culture container in the step of first culturing a stem cell step, and feed (store) a new culture liquid 23 into the culture container. Those in charge take the third flat culture container out of the specimen holder of the electron microscope 16, and discharge the culture liquid 23 fed into the culture container in the step of first fixing a stem cell from the culture container, using an injector or a pipette, and feed (store) a new culture liquid 23 into the culture container, using an injector or a pipette.

Those in charge maintain the third flat culture container at approximately the same temperature as the body temperature of (approx. 37°C), allow it to statically stand for 36 to 48 hours (statically without movement), observe the total area of the stem cell 38 fixed to the bottom of the culture container in relation to the bottom-surface area of the third flat culture container with the electron microscope 13 at an interval of approx. 1 to 2 hours for the period of 36 to 48 hours, and determine whether the total area of the stem cell 38 reaches a target ratio (third target ratio) of the bottom-surface area of the culture container or not. The third target ratio of the total area of the stem cell 38 is 70 to 80% (70 to 80% confluent) of the bottom-surface area of the culture container.

Those in charge, after feeding a new culture liquid 23 into the culture container, click a first culture observation button, and place (set) the culture container in a specimen holder 39 of an electron microscope 13. A spacer 42 is positioned between an upper surface 40 of the specimen holder 39 of an electron microscope 16 and a bottom portion of the third flat culture container so as to keep the bottom portion of the third flat culture container raised by the spacer 42, allow the bottom portion of the third flat culture container and the top portion (feed hole) of the third flat culture container to stay at higher and lower positions, respectively, and maintain the third flat culture container at a predetermined tilted angle (see FIG. 3). Also, a spacer 42 may be positioned between the upper surface 40 of the specimen holder 39 of the electron microscope 16 and the top portion of the third flat culture container so as to keep the top portion of the third flat culture container raised by the spacer 42, allow the top portion of the third flat culture container and the bottom portion of the third flat culture container to stay at higher and lower positions, respectively, and maintain the third flat culture container at a predetermined tilted angle. The tilted angle α1 of the third flat culture container in relation to the upper surface 40 of the specimen holder 39 ranges from 2 to 5°, preferably 2 to 3°.

The method for producing a culture product liquid, after confirming the fixation of the stem cell 38, discharges the culture liquid 23 in the third flat culture container and feed a new culture liquid 23 into the third flat culture container, thereby assuredly encouraging the proliferation of the stem cell 38. The method for producing a culture product liquid places the third flat culture container in relation to the upper surface 40 of the specimen holder 39 at the tilted angle, thereby allowing the stem cell 38 and the culture liquid 23 in the third flat culture container to tilt to the side of the top portion 43 of the third flat culture container (or the side of the bottom portion 41), causing the water pressure of the stem cell 38 and the culture liquid 23 to increase on the side of the top portion 43 of the third flat culture container (or side of the bottom portion 41), allowing the stem cell 38 to concentrate on the side of the bottom portion 41 of the third flat culture container (or side of the top portion 43), and thus increasing the activity of the stem cells 38 to readily and immediately fix the stem cell 38 on the bottom 25 of the third flat culture container.

The display 17 displays a stem cell first culture observation under-way message and a stem cell first culture end button. Those in charge, after confirming the fixation of the stem cell, discharge the culture liquid 23 from the third flat culture container, and feed a new culture liquid 23 into the culture container to assuredly encourage the proliferation of the stem cell 38.

The electron microscope 13 images a magnified view of a plane shape of the stem cell 38 of the third flat culture container at an interval of approx. 1 to 2 hours, and transmits the magnified view of the plane shape of the stem cell 38 to the computer 11 at an interval of approx. 1 to 2 hours. The interval of imaging or image transmission in the electron microscope 13 can be set at 1 to 2 hours by input devices such as a keyboard 14 and a mouse 15. The computer 11 stores a magnified view of a plane shape of the stem cell 38 transmitted from the electron microscope 13 and an imaging time in a storage area so as to be associated with a donor identifier and a stem cell identifier. The computer 11 displays the magnified view of the plane shape of the stem cell 38 transmitted from the electron microscope 13 and the imaging time on the display 17.

Those in charge confirm (visually recognize) the magnified view of the plane shape of the stem cell 38 displayed on the display 17 at an interval of approx. 1 to 2 hours for 36 to 48 hours, and observe the total area of the stem cell 38 (first stem cell) fixed to the bottom of the third flat culture container in relation to the bottom-surface area of the culture container, and determine whether the total area of the stem cell 38 reaches a target ratio (third target ratio) (70 to 80% confluent) of the bottom-surface area of the culture container or not. Those in charge may directly observe the total area of the stem cell 38 in relation to the bottom-surface area of the culture container from the observation window of the electron microscope 13 at an interval of approx. 1 to 2 hours for 36 to 48 hours, and determine whether the total area of the stem cell 38 reaches a target ratio (70 to 80% confluent) of the bottom-surface area of the culture container or not.

Those in charge, according to the observation in the step of first culturing a stem cell, as illustrated in FIG. 16, when the total area of the stem cell 38 (first stem cell) displayed on the display 17 does not reach a target ratio (third target ratio) (70 to 80% confluent) of the bottom-surface area of the third flat culture container, continuously observe the total area of the stem cell 38 in relation to the bottom-surface area of the culture container at an interval of approx. 1 to 2 hours. When the total area of the stem cell 38 reaches a target ratio (third target ratio) of the total area of the magnified view displayed on the display 17, it is determined that the total area of the stem cell 38 reaches a target ratio of the bottom-surface area of the third flat culture container.

According to the observation in the step of first culturing a stem cell, as illustrated in FIG. 17, the stem cell 38 is proliferated on the bottom of the third flat culture container (inner surface of bottom wall) to allow the stem cell to form a colony and its plane shape to expand, and when the total area of the stem cell 38 displayed on display 17 reaches a target ratio (third target ratio) (70 to 80% confluent) of the bottom-surface area of the culture container, the mesenchymal stem cell 38 proliferated (differentiated) from the third flat culture container is extracted.

Those in charge, after confirming that the total area of the mesenchymal stem cell 38 (mesenchymal first stem cell) reaches a target ratio (third target ratio) of the bottom-surface area of the third flat culture container, perform a step of second fixing a stem cell. Those in charge, after confirming that the total area of the mesenchymal stem cell 38 (mesenchymal first stem cell) reaches a target ratio (third target ratio) of the bottom-surface area of the third flat culture container, click a stem cell first culture end button displayed on the display 17. When the stem cell first culture end button is clicked, the computer 11 displays a stem cell first culture end button and a stem cell first extraction end button on the display 17.

Those in charge discharge the culture liquid 23 fed into the third flat culture container from the culture container, and after washing the culture container with PBS, feed a trypsin solution into the culture container. When the trypsin solution is fed into the third flat culture container, the mesenchymal stem cell 38 (first stem cell) fixed to the bottom of the culture container peels off the bottom by the trypsin solution, floating up to the surface of the trypsin solution. Those in charge suck the stem cell 38 using a pipette, and store the stem cell 38 in the pipette.

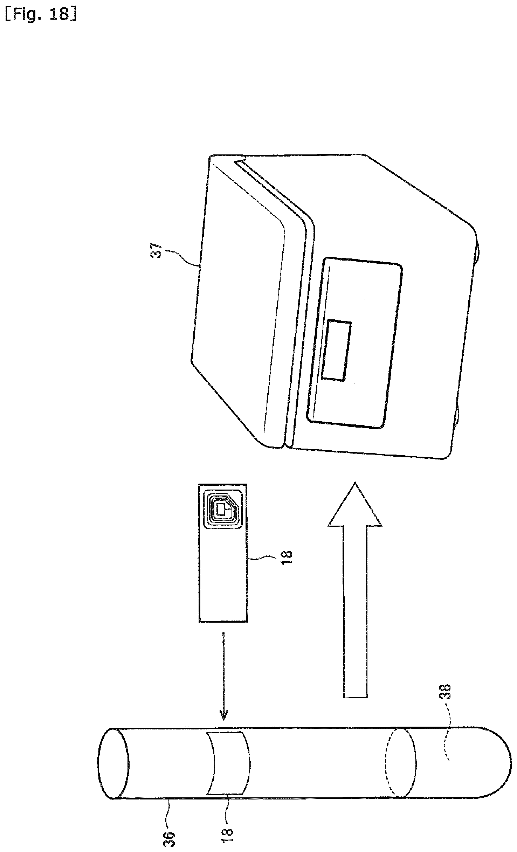

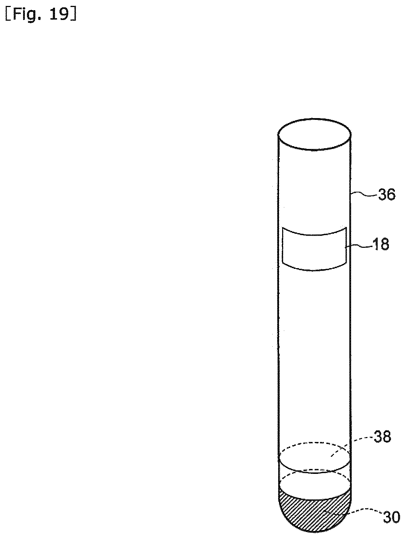

FIG. 18 is a perspective view illustrating a glass test tube 36 and a centrifugal separator 37 used in a step of second fixing a stem cell, and FIG. 19 is a perspective view illustrating a glass test tube 36 after centrifugal separation. A step of extracting a stem cell 38 (first stem cell) from the third flat culture container is followed by a step of second fixing a stem cell. In the step of second fixing a stem cell, the stem cell 38 extracted is centrifugally separated in layers by a centrifugal separator 37. Those in charge such as doctors, nurses, and researchers, after sucking the stem cell 38 from the third flat culture container into the pipette, click a stem cell first extraction end button displayed on the display 17. The display 17 displays a stem cell separation under-way message and a stem cell separation end button.

Those in charge feed (store) the stem cell 38 (first stem cell) in a pipette into its glass test tube 36, and place (set) the glass test tube 36 in the centrifugal separator 37. Those in charge, after centrifugally separating the stem cell 38 for a predetermined period of time with the centrifugal separator 37, take the glass test tube 36 out of the centrifugal separator 37. The stem cell 38 in the glass test tube 36 is vertically separated in layers by the centrifugal separator 37.

After the stem cell 38 (first stem cell) is separated in layers, the mesenchymal stem cell 30 (second stem cell) positioned in the lower layer (lowermost layer) of the stem cell 38 separated in layers is extracted. The stem cell 38 (first stem cell) containing undesired stem cell is centrifugally separated with the centrifugal separator 37 to be vertically separated in layers, and the stem cell 30 (second stem cell) positioned in the lowermost layer of the stem cell 38 centrifugally separated in layers is extracted, thereby assuredly extracting a specific stem cell 30 from the stem cell 38 and removing undesired stem cell from the stem cell 38.

The method for producing a culture product liquid gradually reduces the activity of the stem cell 38 when the total area of the stem cell 38 in relation to the bottom-surface area of the third flat culture container (third culture container) exceeds 80% to proliferate the stem cell 38, and extracts the stem cell 38 from the culture container when the total area of the stem cell 38 in relation to the bottom-surface area of the culture container increases to 70 to 80% for proliferation, thereby maintaining the activity of the stem cell 38, accordingly proliferating the stem cell 38, and extracting the stem cell 30 (second stem cell) having the activity from the stem cell 38.

Those in charge, after vertically separating in layers the stem cell 38 (first stem cell) with the centrifugal separator 37, takes the glass test tube 36 out of the centrifugal separator 37, and click a stem cell separation end button displayed on the display 17. The display 17 displays a stem cell separation end message and a data storing button. Those in charge prepare a fourth flat culture container (fourth culture container) (not shown), affix an IC tag 18 on the outer peripheral surface of the fourth flat culture container, then click a data storing button, and using an IC tag reader/writer 12, store in its IC tag donor data and stem cell data of the IC tag affixed to the third flat culture container. The computer 11 displays a data storing end message, a stem cell second fixing observation button, and a stem cell second fixing end button on the display 17.

A fourth flat culture container used in a step of second fixing a stem cell and a step of second culturing a stem cell is the same (in shape and size) as the second flat culture container 28 (second culture container) used in the step of fixing an activated stem cell and the step of culturing an activated stem cell (see FIG. 6). The fourth flat culture container has a capacity of approx. 40 to 60cc (preferably 50cc), and a bottom-surface area of approx. 50 to 72mm2. The culture container 28 has a side 7 to 8.5mm long.

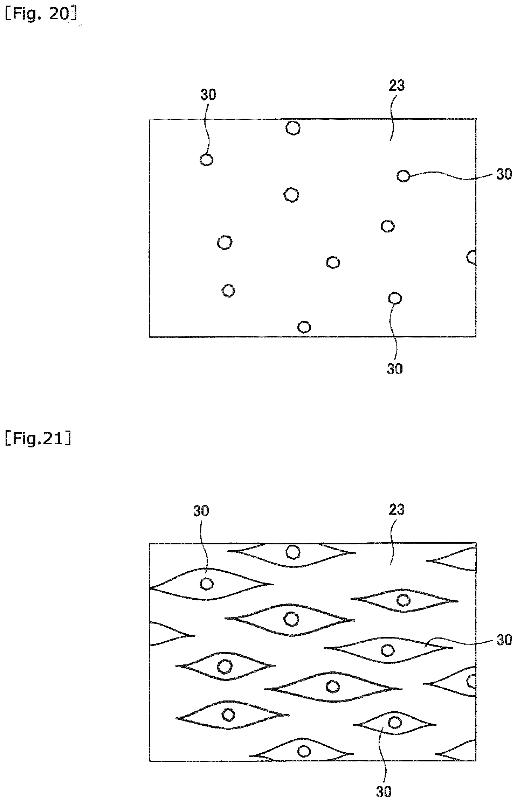

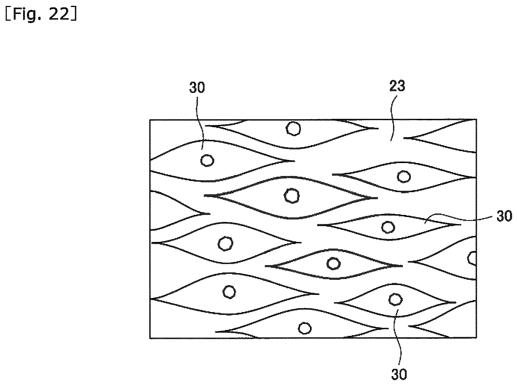

FIG. 20 is a partially enlarged view illustrating one example of a plane shape of a stem cell 30 (second stem cell). FIG. 21 is a partially enlarged view illustrating another example of a plane shape of a stem cell 30 (second stem cell). FIG. 22 is a partially enlarged view illustrating another example of a plane shape of a stem cell 30 (second stem cell). FIGS. 20 to 22 illustrate magnified views of a plane shape of a stem cell 30 (second stem cell) imaged with an electron microscope 13.

Those in charge such as doctors, nurses, and researchers, after extracting a mesenchymal stem cell 30 (second stem cell) present in the lower layer (lowermost layer) of a stem cell 38 (first stem cell) separated in layers in a glass test tube 36, using an injector or a pipette, then feed (store) its stem cell 30 and a culture liquid 23 into a fourth flat culture container (fourth culture container), maintain the culture container at the same temperature as the body temperature (approx. 37°C), allow it to statistically stand for 36 to 48 hours (statistically without movement), observe the deformation of the stem cell 30 (second stem cell) in the culture container from the initial plane shape with an electron microscope 13 at an interval of approx. 1 to 2 hours for the period of 36 to 48 hours, and determine whether the stem cell 30 is fixed to the bottom of the culture container or not.

Those in charge click a stem cell second fixing observation button, and place (set) the fourth flat culture container in a specimen holder of the electron microscope 13. A spacer 42 is positioned between an upper surface 40 of a specimen holder 39 of the electron microscope 13 and a bottom portion of the fourth flat culture container so as to keep the bottom portion of the forth flat culture container raised by the spacer 42, allow the bottom portion of the forth flat culture container and the top portion (feed hole) of the fourth flat culture container to stay at higher and lower positions, respectively, and maintain the fourth flat culture container at a predetermined tilted angle. Also, a spacer 42 may be positioned between the upper surface 40 of the specimen holder 39 of the electron microscope 13 and the top portion of the fourth flat culture container so as to keep the top portion of the fourth flat culture container raised by the spacer 42, allow the top portion of the fourth flat culture container and the bottom portion of the fourth flat culture container to stay at higher and lower positions, respectively, and maintain the fourth flat culture container at a predetermined tilted angle. The tilted angle α2 of the fourth flat culture container in relation to the upper surface 40 of the specimen holder 39 ranges from 2 to 5°, preferably 2 to 3°.

The method for producing a culture product liquid places the fourth flat culture container in relation to the upper surface 40 of the specimen holder 39 at the tilted angle, thereby allowing the stem cell 30 and the culture liquid 23 in the fourth flat culture container to tilt to the side of the top portion of the fourth flat culture container (or the side of the bottom portion), causing the water pressure of the stem cell 30 and the culture liquid 23 to increase on the side of the top portion of the fourth flat culture container (or side of the bottom portion), allowing the stem cell 30 to concentrate on the side of the top portion of the fourth flat culture container (or side of the bottom portion), and thus increasing the activity of the stem cells 30 to readily and immediately fix the stem cell 30 on the bottom of the fourth flat culture container.

The display 17 displays a stem cell second fixing observation under-way message and a stem cell second fixing end button. The electron microscope 13 images a magnified view of a plane shape of the stem cell 30 (second stem cell) fed into the fourth flat culture container at an interval of approx. 1 to 2 hours, and transmits the magnified view of the plane shape of the stem cell 30 to the computer 11 at an interval of approx. 1 to 2 hours. The interval of imaging or image transmission in the electron microscope 13 can be set at 1 to 2 hours by input devices such as a keyboard 14 and a mouse 15. The mesenchymal stem cell 30 (second stem cell) fed into the fourth flat culture container is fixed to the bottom of the culture container as time elapses, cultured by the culture liquid 23, and gradually proliferated (differentiated) on the bottom of the culture container to form a colony.

The computer 11 stores a magnified view of a plane shape of the stem cell 30 (second stem cell) transmitted from the electron microscope 13 and an imaging time in a storage area so as to be associated with a donor identifier and a stem cell identifier. The computer 11 displays the magnified view of the plane shape of the stem cell 30 transmitted from the electron microscope 13 and the imaging time on the display 17. Those in charge confirm (visually recognize) the magnified view of the plane shape of the stem cell 30 displayed on the display 17 at an interval of approx. 1 to 2 hours for 36 to 48 hours, and observe changes in the plane shape of the stem cell 30. Those in charge may directly observe changes in the plane shape of the stem cell 30 from the observation window of the electron microscope 13 at an interval of approx. 1 to 2 hours for 36 to 48 hours.

The initial plane shape of the stem cell 30 (second stem cell) is an approximately circular shape, and when the plane shape of the stem cell 30 is the approximately circular shape, the stem cell 30 is not fixed to the bottom of the fourth flat culture container (inner surface of bottom wall), which does not allow the stem cell 30 to start proliferation (differentiation). The plane shape of the stem cell 30 (second stem cell) deformed is primarily the approximately circular shape before being fixed, and a flat shape when the stem cell 30 elongates (expands) in amorphous shape in one direction (predetermined direction), thereby fixing the stem cell 30 to the bottom of the fourth flat culture container (inner surface of bottom wall) and allowing the stem cell 30 to start proliferation (activation).

Those in charge, according to the observation in the step of second fixing a stem cell, as illustrated in FIG. 20, when the magnified view of the plane shape of the stem cell 30 (second stem cell) displayed on the display 17 remains observed as an approximately circular shape, determine that the stem cell 30 is not fixed to the bottom of the fourth flat culture container (inner surface of bottom wall), and continuously observe changes in the plane shape of the stem cell 30 at an interval of approx. 1 to 2 hours. Those in charge, as illustrated in FIG. 21, when the plane shape of the stem cell 30 (second stem cell) displayed on the display 17 is deformed from the approximately circular to the primarily approximately circular and amorphous flat shape, determine that the stem cell 30 is fixed to the bottom of the culture container.

The use of a large culture container in which the capacity exceeds 60cc and the bottom-surface area exceeds 72mm² when the stem cell 30 (second stem cell) is fixed fails to readily fix the stem cell 30 to the bottom surface of the container and slows down the proliferation of the stem cell 30, but the method for producing a culture product liquid, using the fourth flat culture container having the capacity and the bottom-surface area, can readily fix the stem cell 30 to the bottom of the culture container and immediately proliferate the stem cell 30 in the culture container. The method for producing a culture product liquid allows the fourth flat culture container to statically stand at approximately the same temperature as the body temperature for a period of 36 to 48 hours, and observes the deformation of the stem cell 30 (second stem cell) from the initial plane shape in the culture container at an interval of approx. 1 to 2 hours for the period of 36 to 48 hours, thereby never failing to confirm deformation of the stem cell 30 and properly confirming the fixation of the stem cell 30 to the bottom of the culture container.

According to the observation in the step of second fixing a stem cell step, as illustrated in FIG. 22, the mesenchymal stem cell 30 (second stem cell) is deformed from the approximately circular (initial plane shape) to the primarily approximately circular and amorphous flat shape, and a step of confirming the fixation of the stem cell 30 to the bottom of the fourth flat culture container is followed by a step of second culturing a stem cell. Those in charge such as doctors, nurses, and researchers, after confirming the fixation of the stem cell 30 to the bottom of the fourth flat culture container, click a stem cell second fixing end button displayed on the display 17. When the stem cell second fixing end button is clicked, the computer 11 displays a stem cell second fixing end message, a stem cell second culture observation button, and a stem cell second culture end button on the display 17.

Those in charge discharge the culture liquid 23 fed into the fourth flat culture container from the culture container in the step of second culturing a stem cell step, and feed (store) a new culture liquid 23 into the culture container. Those in charge take the fourth flat culture container out of a specimen holder of an electron microscope 16, and discharge the culture liquid 23 fed into the culture container in the step of first fixing a stem cell from the culture container, using an injector or a pipette, and feed (store) a new culture liquid 23 into the culture container, using an injector or a pipette.

Those in charge maintain the fourth flat culture container at approximately the same temperature as the body temperature of (approx. 37°C), allow it to statically stand for 36 to 48 hours (statically without movement), observe the total area of the stem cell 30 fixed to the bottom of the culture container in relation to the bottom-surface area of the culture container with an electron microscope 13 at an interval of approx. 1 to 2 hours for the period of 36 to 48 hours, and determine whether the total area of the stem cell 28 reaches a target ratio (fourth target ratio) of the bottom-surface area of the culture container or not. The fourth target ratio of the total area of the stem cell 30 is 88 to 92% (88 to 92% confluent) of the bottom-surface area of the culture container.

Those in charge, after feeding a new culture liquid 23 into the fourth flat culture container, click a second culture observation button, and place (set) the culture container in a specimen holder of the electron microscope 13. A spacer 42 is positioned between an upper surface 40 of the specimen holder 39 of an electron microscope 16 and a bottom portion of the fourth flat culture container so as to keep the bottom portion of the fourth flat culture container raised by the spacer 42, allow the bottom portion of the fourth flat culture container and the top portion (feed hole) of the fourth flat culture container to stay at higher and lower positions, respectively, and maintain the fourth flat culture container at a predetermined tilted angle (see FIG. 8). Also, a spacer 42 may be positioned between the upper surface 40 of the specimen holder 39 of the electron microscope 16 and the top portion of the fourth flat culture container so as to keep the top portion of the fourth flat culture container raised by the spacer 42, allow the top portion of the fourth flat culture container and the bottom portion of the fourth flat culture container to stay at higher and lower positions, respectively, and maintain the fourth flat culture container at a predetermined tilted angle. The tilted angle α2 of the fourth flat culture container in relation to the upper surface 40 of the specimen holder 39 ranges from 2 to 5°, preferably 2 to 3°.

The method for producing a culture product liquid, after confirming the fixation of the stem cell 30, discharges the culture liquid 23 in the fourth flat culture container and feed a new culture liquid 23 into the fourth flat culture container, thereby assuredly encouraging the proliferation of the stem cell 30. The method for producing a culture product liquid places the fourth flat culture container in relation to the upper surface 40 of the specimen holder 39 at the tilted angle, thereby allowing the stem cell 30 and the culture liquid 23 in the fourth flat culture container to tilt to the side of the top portion of the fourth flat culture container (or the side of the bottom portion), causing the water pressure of the stem cell 30 and the culture liquid 23 to increase on the side of the top portion of the fourth flat culture container (or side of the bottom portion), allowing the stem cell 30 to concentrate on the side of the bottom portion of the fourth flat culture container (or side of the top portion), and thus increasing the activity of the stem cells 30 to readily and immediately fix the stem cell 30 on the bottom of the fourth flat culture container.

The display 17 displays a stem cell second culture observation under-way message and a stem cell second culture end button. Those in charge, after confirming the fixation of the stem cell 30, discharge the culture liquid 23 from the culture container, feed a new culture liquid 23 into the culture container to assuredly encourage the proliferation of the stem cell 30 (second stem cell).

The electron microscope 13 images a magnified view of a plane shape of the stem cell 30 of the fourth flat culture container at an interval of approx. 1 to 2 hours, and transmits the magnified view of the plane shape of the stem cell 30 to the computer 11 at an interval of approx. 1 to 2 hours. The interval of imaging or image transmission in the electron microscope 13 can be set at 1 to 2 hours by input devices such as a keyboard 14 and a mouse 15. The computer 11 stores a magnified view of a plane shape of the stem cell 30 (second stem cell) transmitted from the electron microscope 13 and an imaging time in a storage area so as to be associated with a donor identifier and a stem cell identifier. The computer 11 displays the magnified view of the plane shape of the stem cell 30 transmitted from the electron microscope 13 and the imaging time on the display 17.

Those in charge confirm (visually recognize) the magnified view of the plane shape of the stem cell 30 displayed on the display 17 at an interval of approx. 1 to 2 hours for 36 to 48 hours, and observe the total area of the stem cell 30 (second stem cell) fixed to the bottom of the fourth flat culture container in relation to the bottom-surface area of the culture container, and determine whether the total area of the stem cell 30 reaches a target ratio (fourth target ratio) (88 to 92% confluent) of the bottom-surface area of the culture container or not. Those in charge may directly observe the total area of the stem cell 30 in relation to the bottom-surface area of the culture container from the observation window of the electron microscope 13 at an interval of approx. 1 to 2 hours for 36 to 48 hours, and determine whether the total area of the stem cell 30 reaches a target ratio (88 to 92% confluent) of the bottom-surface area of the culture container or not.

Those in charge, according to the observation in the step of second culturing a stem cell, as illustrated in FIG. 21, when the total area of the stem cell 30 (second stem cell) displayed on the display 17 does not reach a target ratio (fourth target ratio) (88 to 92% confluent) of the bottom-surface area of the fourth flat culture container, continuously observe the total area of the stem cell 30 in relation to the bottom-surface area of the culture container at an interval of approx. 1 to 2 hours. When the total area of the stem cell 30 reaches a target ratio (fourth target ratio) of the total area of the magnified view displayed on the display 17, it is determined that the total area of the stem cell 30 reaches a target ratio of the bottom-surface area of the fourth flat culture container.

According to the observation in the step of second culturing a stem cell, as illustrated in FIG. 22, the stem cell 30 of the fourth flat culture container (second stem cell) is proliferated on the bottom (inner surface of bottom wall) to allow the stem cell 30 to form a colony and its plane shape to expand, and when the total area of the stem cell 30 displayed on display 17 reaches a target ratio (fourth target ratio) (88 to 92% confluent) of the bottom-surface area of the culture container, a single type of mesenchymal stem cell 30 proliferated (differentiated) from the fourth flat culture container is extracted.

The (residual) culture liquid 23 stored in the fourth flat culture container contains a predetermined metabolite secreted from its stem cell 30 in the process of culturing (proliferating) a single type of mesenchymal stem cell 30 (second stem cell), and the culture liquid 23 that is residual in the fourth flat culture container is a culture product liquid 24. Those in charge, after confirming that the total area of the stem cell 30 (second stem cell) reaches a target ratio (fourth target ratio) of the bottom-surface area of the fourth flat culture container, click a stem cell second culture end button displayed on the display 17. When the stem cell second culture end button is clicked, the computer 11 displays a stem cell second culture end button and a stem cell second extraction end button on the display 17.

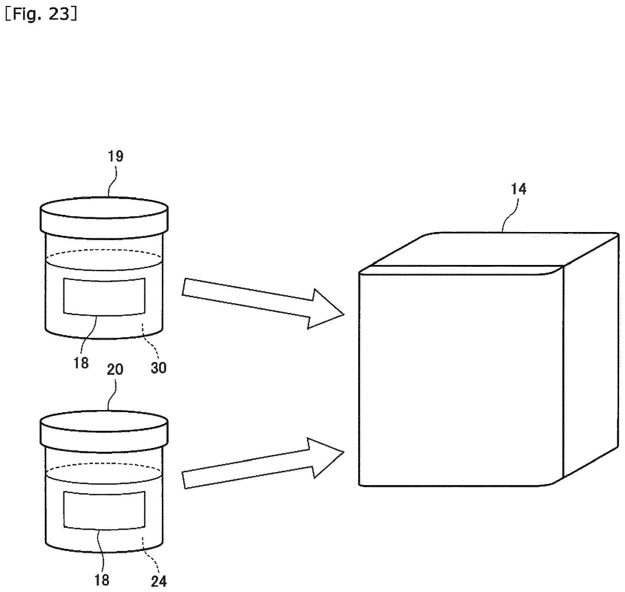

FIG. 23 is a diagram illustrating one example of a stem cell 30 (second stem cell) and a culture product liquid 24 stored. Those in charge suck the culture product liquid 24 (culture liquid 23) fed into the fourth flat culture container from the culture container, using a pipette, and store the culture product liquid 24 in the pipette. Then, those in charge, after washing the fourth flat culture container with PBS, feed a trypsin solution into the culture container. When the trypsin solution is fed into the fourth flat culture container, the mesenchymal stem cell 30 (second stem cell) fixed to the bottom of the culture container peels off the bottom by the trypsin solution, floating up to the surface of the trypsin solution. Those in charge, after sucking the stem cell 30 using a pipette, and storing the stem cell 30 in the pipette, click a stem cell second extraction end button. When the stem cell second extraction end button is clicked, the display 17 displays a stem cell storing container data storing button and a product liquid storing container data storing button.

Those in charge, after extracting the culture product liquid 24 and the stem cell 30 (second stem cell), prepare a stem cell storing container 19 and a product liquid storing container 20, affix an IC tag 18 on the outer peripheral surface of its stem cell storing container 19, and affix an IC tag 18 on the outer peripheral surface of its product liquid storing container 20. Those in charge click the stem cell storing container data storing button, and using an IC tag reader/writer 12, store in the IC tag 18 of the stem cell storing container 19 donor data and stem cell data of the IC tag 18 affixed to the fourth flat culture container. Further, those in charge click the product liquid storing container data storing button, and using an IC tag reader/writer 12, store in the IC tag 18 of the product liquid storing container 20 donor data and stem cell data of the IC tag 18 affixed to the fourth flat culture container. After each of the data is stored in the IC tag 18 of each of the containers 19, 20, the display 17 displays an initial screen.

Those in charge feed (store) the stem cell 30 (second stem cell) into the stem cell storing container 19 from the pipette. A single type of mesenchymal stem cell 30 fed into the stem cell storing container 19 is a single and specific type of mesenchymal stem cell to be cultured having activity obtained by removing undesired mesenchymal stem cell. Those in charge, after feeding the stem cell 30 (a single type of mesenchymal stem cell) into the stem cell storing container 19 from the pipette, store its stem cell storing container 19 in a refrigerator 14 or a freezer 14. The single type of mesenchymal stem cell 30 (second stem cell) is placed in the stem cell storing container 19 and stored in the refrigerator 14 or the freezer 14 at a predetermined temperature (3 to 5°C or refrigerated) for a predetermined period of time.

Those in charge feed (store) the culture product liquid 24 into the product liquid storing container 20 from the pipette. The culture product liquid 24 fed into the product liquid storing container 20 contains a predetermined metabolite secreted from a single and specific type of mesenchymal stem cell 30 to be cultured having activity obtained by removing undesired mesenchymal stem cell. Those in charge, after feeding the culture product liquid 24 into the product liquid storing container 20 from the pipette, store its product liquid storing container 20 in a refrigerator 14 or a freezer 14. The culture product liquid 24 is placed in the product liquid storing container 20 and stored in the refrigerator 14 or the freezer 14 at a predetermined temperature (3 to 5°C or refrigerated) for a predetermined period of time.

### EXPLANATIONS OF LETTERS OR NUMERALS

10 Activated stem cell culturing system
11 Computer
12 IC tag reader/writer
13 Electron microscope
14 Refrigerator or freezer
15 Keyboard
16 Mouse
17 Display
18 IC tag
19 Stem cell storing container
20 Product liquid storing container
21 First flat culture container (first culture container)
22 Dormant stem cell (mesenchymal dormant stem cell)
23 Culture liquid
24 Culture product liquid
25 Bottom
26 Mixed culture liquid
27 Activated stem cell (mesenchymal activated stem cell)
28 Second flat culture container (second culture container)
29 Bottom
30 Stem cell (mesenchymal second stem cell)
31 Raw bone marrow liquid
32 Glass test tube
33 Test tube rack
34 Constant temperature bath
35 Intermediate-layer bone marrow liquid
36 Glass test tube
37 Centrifugal separator
38 Stem cell (mesenchymal first stem cell)
39 Specimen holder
40 Upper surface
41 Bottom portion
42 Spacer
43 Top portion
44 Feed hole
45 Bottom portion
46 Top portion
47 Feed hole

## Claims

1. A method for producing activated stem cells for preparing a single type of activated stem cell by activating a single type of dormant stem cell in a dormant state after storing for a predetermined period of time a single type of stem cell prepared by culturing a bone marrow liquid collected from a donor, the method comprising the steps of:
fixing a dormant stem cell for feeding the single type of dormant stem cell, a predetermined culture liquid, and a culture product liquid prepared in the process of culturing the single type of stem cell before storing the dormant stem cell into a first culture container having a predetermined capacity and a bottom surface of a predetermined area, and fixing the dormant stem cell to the bottom of the first culture container; and
culturing a dormant stem cell for culturing the dormant stem cell fixed to the bottom of the first culture container by the step of fixing a dormant stem cell, proliferating and activating the dormant stem cell until the total area of the dormant stem cell reaches a first target ratio of the bottom-surface area of the first culture container, and transforming the dormant stem cell into the single type of activated stem cell.

2. The method for producing activated stem cells according to claim 1, wherein in the step of culturing a dormant stem cell, a mixed culture liquid of the culture liquid and the culture product liquid is discharged from the first culture container after fixing the dormant stem cell to the bottom of the first culture container by the step of fixing a dormant stem cell, a new culture liquid and a new culture product liquid are fed into the first culture container, and the dormant stem cell fixed to the bottom of the first culture container is cultured, using a new mixed culture liquid of a new culture liquid and a new culture product liquid.

3. The method for producing activated stem cells according to claim 1 or 2, wherein in the step of fixing a dormant stem cell, the first culture container is allowed to statically stand at approximately the same temperature as the body temperature at a predetermined tilted angle for 12 to 24 hours, the deformation of a dormant stem cell from the initial plane shape in the first culture container is observed at an interval of approx. 1 to 2 hours for the period of 12 to 24 hours, and when the dormant stem cell is deformed from the initial plane shape to a predetermined plane shape, it is determined that the dormant stem cell is fixed to the bottom surface of the first culture container.

4. The method for producing activated stem cells according to claim 3, wherein the initial plane shape of the dormant stem cell is an approximately circular shape, and the plane shape of the dormant stem cell deformed is primarily the approximately circular shape, and a flat shape when the dormant stem cell elongates in amorphous form in one direction, and in the step of fixing a dormant stem cell, when the dormant stem cell is deformed to the amorphous flat shape, it is determined that the dormant stem cell is fixed to the bottom surface of the first culture container.

5. The method for producing activated stem cells according to any one of claims 1 to 4, wherein the first target ratio of the total area of the dormant stem cell is 70 to 80% of the bottom-surface area of the first culture container, and in the step of culturing a dormant stem cell, the first culture container is allowed to statically stand at approximately the same temperature as the body temperature at a predetermined tilted angle for 36 to 48 hours, and the total area of the dormant stem cell fixed to the bottom surface of the first culture container in relation to the bottom-surface area of the first culture container is observed at an interval of approx. 1 to 2 hours for the period of 36 to 48 hours.

6. The method for producing activated stem cells according to any one of claims 1 to 5, the method comprising the steps of:
fixing an activated stem cell for extracting from the first culture container the activated stem cell when the total area of the dormant stem cell reaches the first target ratio of the bottom-surface area of the first culture container, feeding the activated stem cell extracted, a new culture liquid, and a new culture product liquid into a second culture container having a predetermined capacity and a bottom surface of a predetermined area, the capacity being larger than the first culture container, and fixing the activated stem cell to the bottom of the second culture container; and
culturing an activated stem cell for culturing the activated stem cell fixed to the bottom of the second culture container by the step of fixing an activated stem cell, and proliferating the activated stem cell until the total area of the activated stem cell reaches a second target ratio of the bottom-surface area of the second culture container.

7. The method for producing activated stem cells according to claim 6, wherein in the step of culturing an activated stem cell, a mixed culture liquid of the culture liquid and the culture product liquid is discharged from the second culture container after fixing the activated stem cell to the bottom of the second culture container by the step of fixing an activated stem cell, a new culture liquid and a new culture product liquid are fed into the second culture container, and the activated stem cell fixed to the bottom of the second culture container is cultured, using a new mixed culture liquid of a new culture liquid and a new culture product liquid.

8. The method for producing activated stem cells according to claim 6 or 7, wherein in the step of fixing an activated stem cell, the second culture container is allowed to statically stand at approximately the same temperature as the body temperature at a predetermined tilted angle for 36 to 48 hours, the deformation of the activated stem cell from the initial plane shape in the second culture container is observed at an interval of approx. 1 to 2 hours for the period of 36 to 48 hours, and when the activated stem cell is deformed from the initial plane shape to a predetermined plane shape, it is determined that the activated stem cell is fixed to the bottom surface of the second culture container.

9. The method for producing activated stem cells according to claim 8, wherein the initial plane shape of the activated stem cell is an approximately circular shape, and the plane shape of the activated stem cell deformed is primarily the approximately circular shape, and a flat shape when the activated stem cell elongates in amorphous form in one direction, and in the step of fixing an activated stem cell, when the activated stem cell is deformed to the amorphous flat shape, it is determined that the activated stem cell is fixed to the bottom surface of the second culture container.

10. The method for producing activated stem cells according to any one of claims 6 to 9, wherein the second target ratio of the total area of the activated stem cell is 88 to 92% of the bottom-surface area of the second culture container, and in the step of culturing an activated stem cell, the second culture container is allowed to statically stand at approximately the same temperature as the body temperature at a predetermined tilted angle for 36 to 48 hours, and the total area of the activated stem cell fixed to the bottom surface of the second culture container in relation to the bottom-surface area of the second culture container is observed at an interval of approx. 1 to 2 hours for the period of 36 to 48 hours.

11. The method for producing activated stem cells according to any one of claims 1 to 10, wherein the culture product liquid, in the process of culturing the single type of stem cell, contains a predetermined metabolite secreted from the single type of stem cell.

12. The method for producing activated stem cells according to any one of claims 1 to 11, wherein the single type of stem cell is prepared by the steps of:
first fixing a stem cell for separating in layers a bone marrow liquid collected from the donor, extracting an intermediate-layer bone marrow liquid positioned in an intermediate layer of the bone marrow liquid separated in layers, feeding the intermediate-layer bone marrow liquid and a predetermined culture liquid into a third culture container having a predetermined capacity and a bottom surface of a predetermined area, and fixing a first stem cell contained in the intermediate-layer bone marrow liquid to the bottom surface of the third culture container;
first culturing a stem cell for discharging the culture liquid in the third culture container after fixing the first stem cell to the bottom surface of the third culture container by the step of first fixing a stem cell, feeding a new culture liquid into the third culture container, culturing the first stem cell, and proliferating the first stem cell until the total area of the first stem cell reaches a third target ratio of the bottom-surface area of the third culture container;
second fixing a stem cell for centrifugally separating in layers the first stem cell cultured by the step of first culturing a stem cell, extracting a second stem cell positioned in the lowermost layer of the first stem cell separated in layers, feeding the second stem cell and a new culture liquid into a fourth culture container having a predetermined capacity and a bottom surface of a predetermined area, the capacity being larger than the third culture container, and fixing the second stem cell to the bottom of the fourth culture container; and
second culturing a stem cell for discharging a culture liquid in the fourth culture container after fixing the second stem cell to the bottom of the fourth culture container by the step of second fixing a stem cell, feeding a new culture liquid into the fourth culture container, culturing the second stem cell, and proliferating the second stem cell until the total area of the second stem cell reaches a fourth target ratio of the bottom-surface area of the fourth culture container.

13. The method for producing activated stem cells according to claim 12, wherein the culture product liquid is a residual culture liquid obtained by extracting the single second stem cell from the fourth culture container.

14. The method for producing activated stem cells according to any one of claims 1 to 13, wherein these stem cells are mesenchymal stem cells.
